(19) Europäisches Patentamt
European Patent Office
Office européen des brevets

(11) EP 4 514 359 B1

(12) # EUROPEAN PATENT SPECIFICATION

(45) Date of publication and mention of the grant of the patent:
**16.04.2025 Bulletin 2025/16**

(21) Application number: **23725087.3**

(22) Date of filing: **25.04.2023**

(51) International Patent Classification (IPC):
***A61K 31/69*** *(2006.01)* ***A61K 9/127*** *(2025.01)*
***A61K 9/19*** *(2006.01)*

(52) Cooperative Patent Classification (CPC):
**A61K 9/19; A61K 9/127; A61K 9/1277; A61K 9/1278;** A61K 31/69

(86) International application number:
**PCT/EP2023/060716**

(87) International publication number:
**WO 2023/208876 (02.11.2023 Gazette 2023/44)**

(54) **NOVEL DUTOGLIPTIN FORMULATIONS AND THEIR PREPARATION**

NEUARTIGE DUTOGLIPTIN-ZUBEREITUNGEN UND IHRE HERSTELLUNG

NOUVELLES FORMULATIONS DE DUTOGLIPTINE ET LEUR PRÉPARATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **26.04.2022 EP 22170010**

(43) Date of publication of application:
**05.03.2025 Bulletin 2025/10**

(73) Proprietors:
- **Recardio Inc.**
  **San Francisco, CA 94105 (US)**
- **Project Pharmaceutics GmbH**
  **82152 Martinsried (DE)**

(72) Inventor: **SCHÜTZ, Andreas**
**82152 Martinsried (DE)**

(74) Representative: **Schiweck Weinzierl Koch Patentanwälte Partnerschaft mbB**
**Ganghoferstraße 68 B**
**80339 München (DE)**

(56) References cited:
**EP-A1- 3 263 134**

- **WPI,, vol. 2004, no. 9, 1 October 2003 (2003-10-01), XP002807983**
- **SAVADI POURIA ET AL: "Piperacillin Encapsulation in Nanoliposomes Using Modified Freeze-Drying of a Monophase Solution Method: Preparation, Characterization and In Vitro Antibacterial Activity", CURRENT MICROBIOLOGY, SPRINGER-VERLAG, NEW YORK, vol. 77, no. 9, 6 May 2020 (2020-05-06), pages 2356 - 2364, XP037213640, ISSN: 0343-8651, [retrieved on 20200506], DOI: 10.1007/S00284-020-02008-0**
- **DATABASE WPI Week 200409, 2004 Derwent World Patents Index; AN 2004-083696, XP002807983**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 514 359 B1

## Description

## Background

Dutogliptin (CAS 852329-66-9 ) is designed as a regenerative medicine to reduce and repair heart muscle injury. It acts as an inhibitor of dipeptidylpeptidase-IV (DPP4) enzyme, which is involved in the degradation of stromal-derived factor-1α (SDF-1α). The drug is given to patients in actual clinical trials as twice-a-day, subcutaneous injection of 60 to 100 mg combined with a subcutaneous dose of filgrastim.

Prior art document EP 3 263 134 A relates to a pharmaceutical composition comprising a DPP-4 inhibitor such as Dutogliptin.

Usually, Dutogliptin is prepared as its tartrate salt. Dutogliptin was originally administered orally. But for acute cardiac indications it needs to be administered parenterally to achieve optimal pharmacokinetic and pharmacodynamic activity. To achieve the required activity with the current formulation it needs to be administered at least twice a day for 2 weeks, resulting patient compliance, acceptance, and injection site issues. Therefore it is desired to reduce the frequency of parenteral administration. It is thus an aim of the present application to deal with such a desire and reducing the number of parenteral administrations, The objective of this invention is thus the provision of preparations of (freeze dried) multilamellar vesicles (MLV) containing encapsulated Dutogliptin and provide liposomal Dutogliptin formulations. These are advantageously suitable for, e.g. subcutaneous (s.c.) or intramuscular (i.m.) application.

## Brief Summary of the invention

1A first aspect refers to a method for the preparation of a liposomal Dutogliptin formulation, comprising the steps:

(B)

B.1) providing an organic phase comprising

(a) one or more phospholipids, wherein the amount of said one or more phospholipids is between 3%(w/w) and 30%(w/w) based on the sum of (a), (b) and (c), preferably 22.36%(w/w) based on the sum of (a), (b) and (c);
(b) one or more organic solvents selected from the group consisting of anisole, ethylacetate, 1,4-dioxane, dimethylcarbonate, dimethylsulfoxide, glycofurol, N,N-dimethylacetamide, N,N-dimethylformamide, N-methyl-2-pyrrolidone (NMP), isopropylideneglycerol, 1-butanol, 2-butanol and tert-butanol or a combination of any of the foregoing; wherein the amount of the said one or more organic solvents is between 60%(w/w) and 97%(w/w) based on the sum of (a), (b) and (c), preferably 74.43%(w/w) based on the sum of (a), (b) and (c);
(c) optionally one or more further organic component(s), which are neither (a) nor (b), wherein the amount of said one or more further organic component(s) is at most 10% based on the sum of (a), (b) and (c);

wherein the sum of (a), (b) and (c) sums up to 100%; and
B.2) providing an aqueous phase comprising

(d) an aqueous medium, wherein the amount of the aqueous medium is between 66%(w/w) and 90%(w/w) based on the sum of (d), (e) and (f), preferably 84%(w/w) based on the sum of (d), (e) and (f);
(e) optionally one or more further components selected from the group consisting of a buffer system, NaOH, HCl, and stabilizers, wherein the amount of all further components is at most 2%(w/w) based on the sum of (d), (e) and (f);
(f) optionally a bulking agent, preferably trehalose, wherein the amount of the bulking agent is at most 10%(w/w) based on the sum of (d), (e) and (f), preferably between 3%(w/w) and 7%(w/w) based on the sum of (d), (e) and (f);

wherein the sum of (d), (e) and (f) sums up to 100%; and
B.3) mixing the aqueous phase and the organic phase until a ratio between 3:1 and 1:3, preferably of 1:1, aqueous phase to organic phase is reached resulting in a nano-dispersed system;
B.4) lyophilize the in a nano-disperse system resulting in a lyophilizate.
B.5) reconstitute the lyophilizate of step B.4 with an aqueous solution resulting in a liposomal Dutogliptin formulation, preferably, wherein the final concentration of Dutogliptin in the liposomal Dutogliptin formulation is between 25 mg/ml and 60 mg/ml, wherein the aqueous solution comprises

(i) 67%(w/w) and 100%(w/w) of an aqueous medium based on the total weight of the aqueous solution, optionally further comprising a tonicity adjusting agent, wherein the amount of the tonicity adjusting agent is at most 20% (w/w) based on the total weight of the aqueous solution optionally further comprising a buffer system,
(ii) Dutogliptin, preferably in form of its tartrate salt or in form of its free base form, wherein the amount of Dutogliptin is between 5%(w/w) and 13%(w/w), more preferably 10±0,5)%(w/w) based on the total weight of the aqueous solution when the Dutogliptin is present in its tartrate salt form or free base form; or (6±0,4)%(w/w) based on the total weight of the aqueous solution when the Dutogliptin is present in its free base form.

One embodiment of aspect 1 or any of its dependent embodiments refers to the method, wherein the organic solvent is tert-butanol (TBA).
Another embodiment of aspect 1 or any of its dependent embodiments refers to the method, wherein the organic phase further comprises cholesterol and wherein the amount of cholesterol is between 2.5%(w/w) and 4%(w/w), preferably 3.2%(w/w).
Another embodiment of aspect 1 or any of its dependent embodiments refers to the method, wherein the organic phase comprises as a phospholipid lecithin and wherein the amount of lecithin is between 18.1%(w/w) and 26.2%(w/w) more preferably 22.13%(w/w).
Another embodiment of aspect 1 or any of its dependent embodiments refers to the method, wherein the organic phase further comprises as phospholipid(s) PG, preferably DOPG-Na, wherein the total amount of PG is between 0,1% (w/w) and 0.4%(w/w), preferably 0.23%(w/w).
Another embodiment of aspect 1 or any of its dependent embodiments refers to the method, wherein the pH of the aqueous phase is between 7 and 7.8, preferably 7.4.
Another embodiment of aspect 1 or any of its dependent embodiments refers to the method, wherein the D90 of the liposomes of the liposomal Dutogliptin formulation resulting from the reconstitution step is between 1 μm and 4.5 μm.
Another embodiment of aspect 1 or any of its dependent embodiments refers to the method, wherein the only solvent of an aqueous solution is water.
Another embodiment of aspect 1 or any of its dependent embodiments refers to the method, wherein the aqueous solution is an aqueous NaCl solution wherein the amount of NaCl is between 8g/l and 10 g/l, preferably between 8.8 g/l and 9.2 g/l, more preferably 9 g/l. Another embodiment of aspect 1 or any of its dependent embodiments refers to the method, wherein the aqueous solution in Step B.5 is a Dutogliptin tartrate solution.
Another embodiment refers to the latter embodiment, wherein the aqueous solution further comprises 9 g/l NaCl.
Another embodiment of aspect 1 or any of its dependent embodiments refers to the method, wherein the total amount of Dutogliptin in the liposomal Dutogliptin formulation is between 60 mg and 100 mg.
Another aspect (aspect 2) refers to a liposomal Dutogliptin formulation comprising

(i) an aqueous medium, preferably water, wherein the amount of the aqueous medium is between 65%(w/w) and 90% (w/w) based on the total weight of the liposomal Dutogliptin formulation;
(ii) a lipid, preferably cholesterol, wherein the amount of said lipid, preferably cholesterol is between 1%(w/w) and 2.5%(w/w) based on the total weight of the liposomal Dutogliptin formulation;
(iii) one or more phospholipid(s), wherein the amount of said one or more phospholipid(s) is between 7%(w/w) and 15%(w/w), preferably (11.2+0.4)(w/w), based on the total weight of the liposomal Dutogliptin formulation, preferably, wherein the one or more phospholipids are PC, PG and DSPG;
(iv) one or more agent(s) selected from the group consisting of glycine, arginine, proline, or any other amino acid known to be suitable as a bulking agent, a saccharide component selected from the group consisting of sucrose, trehalose, arabinose, erythritol, fructose, galactose, glucose, lactose, maltitol, maltose, maltotriose, mannitol, mannobiose, mannose, ribose, sorbitol, xylitol, xylose, dextran, dextrose, and NaCl, wherein the total amount of said one or more agent(s) is between 1.5%(w/w) and 5.5%(w/w);
(v) Dutogliptin, wherein the amount of Dutogliptin, preferably in its tartrate salt form or free base form, is between 3% (w/w) and 12%(w/w), preferably (5.3±0.3) (w/w) based on the total weight of the liposomal Dutogliptin formulation and the final concentration of Dutogliptin in the liposomal Dutogliptin formulation is between 25 mg/ml and 60 mg/ml;

wherein the sum of (i) to (v) sums up to 100%.
Another aspect (aspect 3) refers to a kit comprising a lyophilizate described in aspect 1 and its embodiments in a container and a pharmaceutical aqueous solution described in aspect 1 and its embodiments in a second container.
One embodiment of aspect 3 refers to the kit, further comprising a manual how to combine the content of the first and second container to receive a liposomal Dutogliptin formulation according to the last embodiment of aspect 1; or the liposomal Dutogliptin formulation of aspect 2.

**Definitions**

If not stated otherwise, amounts in % refer to % (weight/weight) ((w/w)).
If not explicitly mentioned otherwise (e.g. by using a term such as "a specific" meaning "one"), the term "a" is an indefinite article encompassing "one" and "one or more" / "more than one" noun(s) following the term "a".
The term "amount of one or more" components such as the amount of one or more organic solvent(s) refers to the total sum of all such components. For example, if two organic solvents are present in an organic phase, e.g. 20%(w/w) based on the total amount of the organic phase tert-butanol and 10%(w/w) based on the total weight of the organic phase propanol the amount of the said one or more organic solvents (the two organic solvents propanol and tert-butanol) is 30% (w/w) based on the total weight of the organic phase.
A "buffer" or "buffer system" as used herein is used to prevent major changes in the pH of a solution, and suitable examples are well-known to the skilled formulator.
A "bulking agent" as used herein and as the name implies, form the bulk of a lyophilized product, and provide an adequate structure to a lyophilized cake. Non limiting examples of bulk agents are mannitol, glycine, arginine, proline, glucose, sucrose, lactose, trehalose, and dextran.
The term "Cholesterol" refers to 3β-Hydroxy-5-cholestene (CAS No.: 57-88-5). Examples of derivatives of cholesterol are cholesteryl sulfate and its salts (e.g., sodium salt), cholesteryl hemisuccinate, cholesteryl succinate, cholesteryl oleate, polyethylene glycol derivatives of cholesterol (cholesterol-PEG), coprostanol, cholestanol, cholestane, cholic acid, cortisol, corticosterone, hydrocortisone and calciferol.
The term "Container" as used herein means an ampoule or vial with rubber stopper and cap, single or double chamber syringe, infusion bag or bottle made from polymeric materials or glass, suitable for housing compositions for parenteral administration. It also includes any vessel for holding liquids.
The term "D90" is well known to a skilled person and refers in connection with size distributions to the number of vesicles with diameters at or below a given value having a weight of 90% of the weight of the components forming such particles in a formulation. The D90 can be determined via multiangle light scattering (MALS).
"Dutogliptin" is a potent, and selective dipeptidyl peptidase-4 (DPP4) inhibitor for the treatment of type 2 diabetes mellitus. It has the following chemical structure:

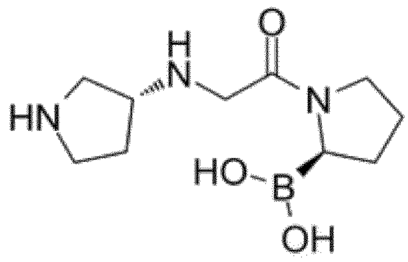

The term Dutogliptin encompasses the molecule as a free base or any of its pharmaceutically acceptable salts such as its tartrate salt or a fatty acid salt.
When calculating concentrations (mg/ml) of Dutogliptin, the concentration is based on the Dutogliptin free base molecule.
"Further organic compound" as used herein refers to organic compound which are no solvents and which are miscible with water, e.g. lipids such as cholesterol, polysaccharides, and polypeptides. The term "lipid" as used herein excludes phospholipid(s). The term lipids as used in the present invention refers to lipids selected from the group consisting of fatty acids, sterols (e.g. cholesterol), fat-soluble vitamins (such as vitamins A, D, E and K), glycerolipids (such as monoglycerides, diglycerides, triglycerides), sphingolipids, saccharolipids, polyketides, isoprenoids (prenol lipids) and waxes.
"Miscible with water" as used herein means an organic solvent can be mixed with water in all proportions, e.g. ethanol, propanol, butanol, respectively with water.
The term "between X% and Y%" (wherein X and Y represent any number between 0 and 100 and X% is smaller than Y%) refers to any number within the range between X% and Y% including X% and Y%.
A "nano-disperse system" as used herein refers to a combination of an organic and an aqueous phase i.e. an aqueous formulation comprising vesicles or micelles with a D90 of 60 nm or less, wherein the vesicles or micelles are no liposomes.
A "liposome" is a spherical vesicle having at least one lipid bilayer. The liposome can be used as a vehicle for administration of pharmaceutical drugs. Liposomes are most often composed of phospholipids, especially phosphatidylcholine, but may also include other lipids, such as egg phosphatidylethanolamine, so long as they are compatible with

lipid bilayer structure.

"Liposomal formulation" as used herein means a liquid comprising liposomes, said liposomes comprising phospholipids. Said liposomal formulation is suitable for encapsulation of Dutogliptin in the aqueous environment of the liposomes.

Liposomal size or lipophilic vesicle size (such as micelle size), respectively, as disclosed herein refers to the size as determined by multiangle light scattering (MALS) or alternatively by dynamic light scattering (DLS). Usually, the size of liposomes is in the range from 0,025 μm to 2,5 μm (Akbarzadehet al. Nanoscale Research Letters 2013,8:102).

A liposomal formulation of the present invention may have the advantage that it reduces the number of injections or intakes of Dutogliptin which a patient requires per each day. Accordingly, it contributes to patient convenience and/or to patient compliance.

"Loading" means incorporating or transferring Dutogliptin into liposomes/encapsulating Dutogliptin with the liposomes.

"Organic component" as used herein refers to any molecule comprising at least one $-(CH_2)-$moiety.

"Pharmaceutically acceptable" means approved or approvable by a regulatory agency in a country or that is listed in the European or U.S. Pharmacopoeia or other generally recognized pharmacopoeia for use in animals, and more particularly, in humans.

PEG means polyethylene glycol.

PEGylation is the process of both covalent and non-covalent attachment or amalgamation of PEG polymer chains to molecules and macrostructures, such as phospholipids, which leads to vesicles then described as PEGylated. PEGylated phospholipids are well known and commercially available.

The "pharmaceutical composition" described herein is, in particular, a pharmaceutical liposomal composition. A "pharmaceutical liposomal composition" means a composition comprising liposomes which is suitable for pharmaceutical administration.

The "phospholipids" used in the formulations of the present invention comprise a hydrophilic phosphate moiety and two hydrophobic carbohydrate moieties. For clarification's sake, phospholipids are not encompassed by the term lipids. The phospholipids can be selected form the group consisting of a natural phospholipid, a synthetic phospholipid, and combinations thereof. Lecithin is one of the natural resources for phospholipid. Lecithin is a mixture found in egg yolk and soya. It comprises a number of phospholipids including phosphatidylcholine (PC), phosphatidylethanolamine (PE), and phosphatidylinositol (PI) or a (pharmaceutically acceptable) salt of any of the foregoing.

Generally, the structure of a phospholipid as used herein is a phospholipid of structure (I)

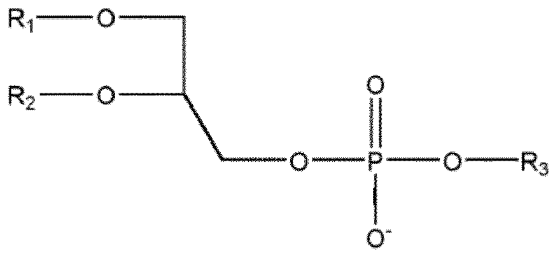

(I)

wherein

$R_1$ represents $C_{10}$-$C_{24}$acyl:

$R_2$ represents $C_{10}$-$C_{24}$acyl or hydrogen;

$R_3$ represents 2-trimethylamino-1-ethyl (resulting in PC), 2-amino-2-carboxy-1ethyl (resulting in PS), inosityl group ($C_6H_{11}O_5$) (resulting in PI), 2-amino-1ethyl (resulting in PE) or hydrogen (resulting in PA).

The terms "phosphatidic acid" and "PA" are used interchangeably herein. PA or a (pharmaceutically acceptable) salt thereof can derive from a natural source and/or a synthetic source. Non-limiting examples for PA are DLPA, DMPA, DPPA, DSPA, POPA, POPA, DEPA, HSPA, HEPA or a (pharmaceutically acceptable) salt of any of the foregoing.

The terms "phosphatidylcholine" and "PC" are used interchangeably herein. PC or a (pharmaceutically acceptable) salt thereof can derive from a natural source and/or a synthetic source. PC can be PEGylated. Non-limiting examples for PC are dilauroylphosphatidylcholine, (DLPC), dimyristoylphosphatidylcholine (DMPC), dipalmitoylphosphatidylcholine (DPPC), distearoylphosphatidylcholine (DSPC), dioleoylphosphatidylcholine (DOPC), palmitoyl-oleoylphosphatidylcholine (POPC), dierucoylglycerophosphocholine (DEPC), hydrogenated soy phosphatidylcholine (HSPC), hydrogenated egg phosphatidylcholine (HEPC) or a (pharmaceutically acceptable) salt of any of the foregoing.

The terms "phosphoethanolamine" and "PE" are used interchangeably herein. PE can be PEGylated. PE or a (pharmaceutically acceptable) salt thereof can derive from a natural source and/or a synthetic source. Non-limiting examples PE are DLPE, DMPE, DPPE, DSPE, POPE, POPE, DEPE, HSPE, HEPE or a (pharmaceutically acceptable) salt of any of the foregoing.

The terms "phosphatidylglycerol" and "PG" are used interchangeably herein. PG or a (pharmaceutically acceptable) salt thereof can derive from a natural source and/or a synthetic source. Non-limiting examples for PG are DLPG, DMPG, DPPG, DSPG, POPG, POPG, DEPG, HSPG, HEPG or a (pharmaceutically acceptable) salt of any of the foregoing.

The terms "phosphatidylinositol" and "PI" are used interchangeably herein. PI can be PEGylated. PI or a (pharmaceutically acceptable) salt thereof can derive from a natural source and/or a synthetic source. Non-limiting examples for PI are DLPI, DMPI, DPPI, DSPI, POPI, POPI, DEPI, HSPI, HEPI or a (pharmaceutically acceptable) salt of any of the foregoing.

The terms "phosphoserine" and "PS" are used interchangeably herein. PS can be PEGylated. PS or a (pharmaceutically acceptable) salt thereof can derive from a natural source and/or a synthetic source. Non-limiting examples for PS are DLPS, DMPS, DPPS, DSPS, POPS, POPS, DEPS, HSPS, HEPS or a (pharmaceutically acceptable) salt of any of the foregoing.

Non-limiting examples for pharmaceutically acceptable salts of any of the phospholipids are sodium salts or ammonium salts such as PG-Na PG-$NH_4$, DSPG-Na or DSPG-$NH_4$.

When using the terms PC, PG, PE, PA, PS and/or PI, these terms also encompass the pharmaceutically acceptable salt form of said phospholipids or PEGylated modifications of said phospholipids, if not otherwise indicated.

The term "reconstitute a lyophilizate" means mixing a lyophilizate with a solution.

Mixing "shortly before administration to patient" means up to three days before, in particular up to 24 hours before, and for example up to 6 hours before administration to the patient.

A "tonicity adjusting agent" means a pharmaceutically acceptable compound which can be added to a formulation to make it isotonic with human plasma. Tonicity adjusting agents include for example dextrose, glucose, mannitol, sucrose, lactose, trehalose, glycerin and NaCl, particularly sucrose or glycerin or NaCl, more particularly sucrose or NaCl. Tonicity is the 'effective osmolality' and is equal to the sum of the concentrations of the solutes which have the capacity to exert an osmotic force across the membrane. Parenteral formulations should be isotonic with blood plasma. Tonicity adjusting agents are well known to the skilled person. The skilled person is aware that in some cases, a tonicity adjusting agent may also have other function(s) such as trehalose being also a bulk agent.

As used herein, the terms "treat", "treating" or "treatment" of any disease or disorder refers in one embodiment, to ameliorating the disease or disorder (i.e., slowing or arresting or reducing the development of the disease or at least one of the clinical symptoms thereof). In another embodiment, "treat", "treating" or "treatment" refer to alleviating or ameliorating at least one physical parameter including those which may not be discernible by the patient. In yet another embodiment, "treat", "treating" or "treatment" refer to modulating the disease or disorder, either physically, (e.g., stabilization of a discernible symptom), physiologically, (e.g., stabilization of a physical parameter), or both.

The term "wherein the sum of a), b), c), etc. (and further enumerations) sums up to 100%" means that the percent values given to the components a) to e) always have to be chosen in a way that the sum of the values for a) to e) will be 100. Thus, these values give a ratio between the components a) to e) . However, the skilled person understands that a formulation may comprise additional components such as a solvent. However, the amount of such an additional component is not considered when calculating the ratios between a) to c) (which values in % always have to sum up to 100%).

It is understood that, as long as a combination does not violate any law of nature, all embodiments of the present invention, irrespective of the fact if an embodiment is an embodiment, a preferred embodiment, a more preferred embodiment, a particularly preferred embodiment or a most preferred embodiment, can be combined and such combinations of two or more embodiments of the invention are disclosed herein by disclosing the two or more embodiments even if the combination of such two or more embodiments is not explicitly referred to.

The skilled person will understand that all embodiments described herein, irrespectively if the embodiments are embodiments, preferred embodiments, more preferred embodiments etc. can be combined unless such a combination contradicts a law of nature. The skilled person will understand that embodiment such as preferred embodiments for one aspect of the present invention can also apply to another aspect. Thus, a preferred embodiment for, e.g. a method according to the invention is also a preferred embodiment - if applicable - for, e.g., a lyophilizate or liposomal Dutogliptin formulation according to the invention.

## Detailed description

The present invention refers to liposomal formulations of Dutogliptin and methods for the preparation of liposomal formulations comprising Dutogliptin.

Thus, one aspect of the present invention refers to a liposomal formulation of Dutogliptin comprising

(i) an aqueous medium, preferably water, wherein the amount of the aqueous medium is between 65% and 90% based on the total weight of the liposomal Dutogliptin formulation;
(ii) a lipid, preferably cholesterol, wherein the amount of said lipid, preferably cholesterol is between 1%(w/w) and 2.5%(w/w) based on the total weight of the liposomal Dutogliptin formulation;
(iii) one or more phospholipid(s), wherein the amount of said one or more phospholipid(s) is between 7%(w/w) and 15%(w/w), preferably (11.2±0,4)(w/w), based on the total weight of the liposomal Dutogliptin formulation;
(iv) one or more agent(s) selected from the group consisting of glycine, arginine, proline, or any other amino acid known to be suitable as a bulking agent, sucrose, trehalose, arabinose, erythritol, fructose, galactose, glucose, lactose, maltitol, maltose, maltotriose, mannitol, mannobiose, mannose, ribose, sorbitol, xylitol, xylose, dextran, dextrose, NaCl, a buffer system, and a stabilizer, wherein the total amount of said one or more agent(s) is between 1.5%(w/w) and 5.,5%(w/w);
(v) Dutogliptin, wherein the amount of Dutogliptin, preferably in its tartrate salt form or free base form, is between 3% (w/w) and 12%(w/w), preferably (5.3±0.3) (w/w) based on the total weight of the liposomal Dutogliptin formulation; and preferably the final concentration of Dutogliptin in the liposomal Dutogliptin formulation is between 25 mg/ml and 60 mg/ml;

wherein the sum of (i) to (v) sums up to 100%.

Another aspect refers to a method for the preparation of a liposomal formulation of Dutogliptin, comprising the steps:

(B)

B.1) providing an organic phase comprising

(a) one or more phospholipids, wherein the amount of said one or more phospholipids is between 3%(w/w) and 30%(w/w) based on the sum of (a), (b) and (c), preferably 22.36%(w/w) based on the sum of (a), (b) and (c);
(b) one or more organic solvents selected from the group consisting of anisole, ethylacetate, 1,4-dioxane, dimethylcarbonate, dimethylsulfoxide, glycofurol, N,N-dimethylacetamide, N,N-dimethylformamide, N-methyl-2-pyrrolidone (NMP), isopropylideneglycerol, 1-butanol, 2-butanol and tert-butanol or a combination of any of the foregoing; wherein the amount of the said one or more organic solvents is between 60%(w/w) and 97%(w/w) based on the sum of (a), (b) and (c), preferably 74.43%(w/w) based on the sum of (a), (b) and (c);
(c) optionally one or more further organic component(s), which are neither (a) nor (b), wherein the amount of said one or more further organic component(s) is at most 10% based on the sum of (a), (b) and (c);

wherein the sum of (a), (b) and (c) sums up to 100%; and
B.2) providing an aqueous phase comprising

(d) an aqueous medium, wherein the amount of the aqueous medium is between 66%(w/w) and 90%(w/w) based on the sum of (d), (e) and (f), preferably 84% based on the sum of (d), (e) and (f);

In one embodiment, in (d) the amount of the aqueous medium is between 88% (w/w) and 100% (w/w) based on the sum of (d), (e) and (f);

In one embodiment in (d) the amount of the aqueous medium is between 88% (w/w) and 99.99 % (w/w) based on the sum of (d), (e) and (f), wherein (f) is at most 10% (w/w) based on the sum of (d), (e) and (f).

In one embodiment in (d) the amount of the aqueous medium is between 91% (w/w) and 96.99 % (w/w) based on the sum of (d), (e) and (f), wherein (f) is between 3%(w/w) and 7%(w/w) based on the sum of (d), (e) and (f).

wherein "99.99" and "96.99" represent the fact that (e) and (f) may be present but the concentration may be very low.

In one embodiment in (d) the amount of the aqueous medium is between 91% (w/w) and 96.5 % (w/w) based on the sum of (d), (e) and (f), wherein (f) is between 3%(w/w) and 7%(w/w) based on the sum of (d), (e) and (f)

and (e) is between 0.5 and 2%(w/w) based on the sum of (d), (e) and (f).

(e) optionally one or more further components selected from the group consisting of a buffer system and pH adjusting acids and/or bases (e.g. NaOH, HCl), wherein the amount of all further components is at most 2%(w/w) based on the sum of (d), (e) and (f);
In one embodiment in (e) the amount of all further components is between 0.5 and 2%(w/w) based on the sum of (d), (e) and (f)
(f) optionally a bulking agent, preferably trehalose, wherein the amount of the bulking agent is at most 10%(w/w) based on the sum of (d), (e) and (f), preferably between 3%(w/w) and 7%(w/w) based on the sum of (d), (e) and (f);

wherein the sum of (d), (e) and (f) sums up to 100%; and
B.3) mixing the aqueous phase and the organic phase until a ratio between 3:1 and 1:3, preferably of 1:1, aqueous phase to organic phase is reached resulting in a nano-dispersed system;
B.4) lyophilize the in a nano-disperse system resulting in a lyophilizate; optionally
B.5) reconstitute the lyophilizate of step B.4 with an aqueous solution resulting in a liposomal Dutogliptin formulation, preferably, wherein the final concentration of Dutogliptin in the liposomal Dutogliptin formulation is between 25 mg/ml and 60 mg/ml, wherein the aqueous solution comprises

(i) 67% (w/w) and 100%(w/w) of an aqueous medium based on the total weight of the aqueous solution, optionally further comprising a tonicity adjusting agent, wherein the amount of the tonicity adjusting agent is at most 20% (w/w), preferably 10% (w/w) to 20% (w/w), based on the total weight of the aqueous solution optionally further comprising a buffer system,
(ii) Dutogliptin, preferably in form of its tartrate salt or in form of its free base form, wherein the amount of Dutogliptin is between 5%(w/w) and 13%(w/w), more preferably 10$\pm$0,5)%(w/w) based on the total weight of the aqueous solution when the Dutogliptin is present in its tartrate salt form; or (6$\pm$0,4)%(w/w) based on the total weight of the aqueous solution when the Dutogliptin is present in its free base form.

The method of the invention allow the straight-forward preparation of a liquid intermediate formulation nano-disperse system) wherein the D90 of the lipophilic vesicles or micelles, is 60 nm or less, more preferably 25 nm or less, preferably 20 nm or less. This nano-dispersed system which is formed during a method according to the invention allows the preparation of a liposomal formulation without the need to mechanically reducing the size of liposomes (e.g. for enabling sterile filtration of the Dutogliptin formulation before filling into sterile containers and/or freeze drying).

Alternatively, the method of the invention further allow the easy preparation of a liposomal formulation, wherein the D90 of the liposomes is between 1 $\mu$m and 4.5 $\mu$m. The present invention further provides a formulation comprising Dutogliptin prepared according to a method of the present invention and more specifically a formulation comprising Dutogliptin suitable for administration to patients. In particular, such administration is by subcutaneous injection or infusion, more preferably by subcutaneous injection. The invention further provides two separate formulations which can be mixed together shortly before administration to the patient, in order to provide the liposomal composition suitable for administration. In one embodiment, one formulation comprising phospholipids may be a lyophilizate, the second formulation may be an aqueous formulation with the proviso that at least either the lyophilizate or the aqueous solution comprises Dutogliptin. When the two separate formulations are mixed together, Dutogliptin is loaded into the forming liposomes and resulting in a pharmaceutical liposomal Dutogliptin formulation suitable for use in the clinic. Preferably, wherein the final concentration of Dutogliptin in the liposomal Dutogliptin formulation is between 25 mg/ml and 60 mg/ml.

The formulation comprising Dutogliptin should enable efficient and optimal loading of Dutogliptin into liposomes before administration to the patient.

In one preferred embodiment, at least 25%(w/w) of Dutogliptin, more preferably at least 30%(w/w) of Dutogliptin are encapsulated by liposomes in a liposomal Dutogliptin formulation.

Preferably, the invention provides a pharmaceutical liposomal formulation which enables an extended release of Dutogliptin from the liposomes after administration (injection).

The formulations described herein are, in particular, pharmaceutical formulations, such as a pharmaceutical liposomal composition.

Preferably, the D90 of liposomes of a liposomal formulation is between 1 $\mu$m and 4.5 $\mu$m.

In particular, a phospholipid or a pharmaceutically acceptable salt thereof as described herein is selected from egg lecithin, soy lecithin, or synthetic phospholipids.

Described below are a number of aspects and embodiments of the invention.

## Formulation

A first aspect refers to a liposomal Dutogliptin formulation as described herein.

The skilled person will understand that traces of solvents and additives used in the preparation method, preferably in a preparation method of the invention described further below (such as tert-butanol, or water, or a buffer system), can still be present up to an amount of up to 2%; or further additives may be present in liposomal Dutogliptin formulation. Without being bound to this explanation, further additives may be required for stabilization purpose or tolerability of such a liposomal Dutogliptin formulation for a patient.

In one preferred embodiment, the combined weight of (i) to (v) is at least 98%(w/w) based on the total weight of a liposomal Dutogliptin formulation.

In another preferred embodiment, the combined weight of (i) to (v) is at least 99%(w/w) based on the total weight of a liposomal Dutogliptin formulation.

In one preferred embodiment, the combined weight of (i) to (v) is at least 99,9%(w/w) based on the total weight of a liposomal Dutogliptin formulation.

One preferred embodiment refers to a liposomal Dutogliptin formulation wherein residues of an organic solvent, such as tert-butanol, is less than 2%(w/w) based on the total weight of such a formulation (e.g. the combined amount of (i) to (v) and tert-butanol). More preferably, the amount of tert-butanol is 1% or less, such as less than 0,8% or even less than 0,01%.

One preferred embodiment refers to a formulation according to the invention, wherein the formulation consists of (i) to (v).

Another preferred embodiment refers to a formulation according to the invention, wherein the formulation consists of (i) to (v) tert-butanol and a buffer system, preferably an acetate buffer system, wherein the combined amount of tert-butanol and the buffer system is between 0,01% and 2% based on the total weight of such a formulation (e.g. the combined amount of a) to e) and tert-butanol, e.g. in case of a lyophilizate).

One further preferred embodiment refers to a, preferably pharmaceutical, nano-disperse system comprising Dutogliptin (or a method comprising the preparation of a nano-dispersed system), wherein the D90 of the lipophilic vesicles is less than 25 nm, more preferably 20 nm or less, such as between 10 nm and 20 nm e.g., around 15 nm, or between 3 nm and 10 nm, e.g., around 5 nm.

Another preferred embodiment refers to a lyophilized formulation comprising Dutogliptin.

Another preferred embodiment refers to a lyophilized formulation (a lyophilizate), wherein the amount of Dutogliptin is between 50 mg and 120 mg, more preferably between 60 mg and 100 mg.

In a further preferred embodiment, the pharmaceutically active substance in the formulation according to the invention is entrapped by a lipid clathrate, proliposome, micelle or liposome, more preferably a liposome or a micelle. In other words, the composition comprises e.g., at least one liposome or at least one micelle, respectively, and Dutogliptin is loaded into the liposome or micelle.

In one preferred embodiment Dutogliptin in the formulation according to the invention is entrapped by a micelle (a nano-dispersed formulation/a nano-disperse system). Most preferably the micelles have a D90 is 60 nm or less, such as less than 25 nm, more preferably 20 nm or less, such as between 10 nm and 20 nm, or between 3 nm and 10 nm.

In another preferred embodiment, Dutogliptin in the formulation according to the invention is entrapped by a liposome (a liposomal formulation).

Another preferred embodiment refers to a formulation wherein the formulation, preferably a liposomal formulation or a nano-dispersed formulation, is a pharmaceutical formulation.

## Liposomal formulation

One preferred embodiment refers to a pharmaceutical liposomal formulation.

Such a pharmaceutical liposomal formulation is preferably suitable for injection, preferably subcutaneous injection.

Another preferred embodiment refers to a pharmaceutical liposomal formulation wherein the concentration of Dutogliptin in a liposomal formulation is between 25 mg/ml and 60 mg/ml such as between 30 mg/ml and 50 mg/ml.

According to another preferred embodiment, the pharmaceutical liposomal formulation has a pH between 6 and 8, preferably between 7 and 8, more preferably between 7 and 7,8 such as 7,4$\pm$2.

## (i) Solvent for liposomal formulation

### Aqueous medium

The aqueous medium of a liposomal formulation is water or a combination of water with a solvent selected from

the group consisting of propylene glycol, ethanol, isopropanol, glycerol, glycol ethers (such as monobutyl ether of ethylenglycol or propyleneglycol, or the monoethylether or the monomethylether of diethyleneglycol or any combination thereof), polyethylenglycol (PEG) 300, PEG 400, in particular propylene glycol and ethanol, wherein the amount of water of the total amount of solvent of the aqueous medium is at least 60%(w/w), preferably at least 90%(w/w), more preferably at least 95%(w/w).

In one preferred embodiment, water is the only solvent in an aqueous medium and, thus, the only solvent of a liposomal formulation.

Generally, a liposomal formulation comprises an aqueous medium, wherein the amount of the aqueous medium is between 65%(w/w) and 90%(w/w) based on the sum of (i) to (v).

**(ii) lipid**

In one preferred embodiment, a formulation according to the invention comprises one or more lipids.

In one preferred embodiment, a lipid as used in the present invention is a lipid selected from the group consisting of fatty acids, sterols (e.g. cholesterol), fat-soluble vitamins (such as vitamins A, D, E and K), glycerolipids (such as monoglycerides, diglycerides, triglycerides), sphingolipids, saccharolipids, polyketides, isoprenoids (prenol lipids) and waxes, more preferably from the group consisting of fatty acids, sterols (e.g. cholesterol), fat-soluble vitamins (such as vitamins A, D, E and K), glycerolipids (such as monoglycerides, diglycerides, triglycerides), sphingolipids, and saccharolipids.

Cholesterol is known to have an influence on the stability of liposomes and on drug release.

Therefore, in one more preferred embodiment, a formulation according to the invention preferably comprises cholesterol or a derivative thereof.

Another preferred embodiment refers to a formulation, wherein the further organic component is selected from the group consisting of cholesterol or sodium cholesteryl sulfate. More preferably, the further organic component is cholesterol.

Another preferred embodiment refers to a formulation, wherein the amount of cholesterol is between 0,5% and 3% compared to the total sum of all components in the formulation. For example, around 1,6% such as between 1,3% and 1,9%.

**(iii) phospholipid(s)**

Another preferred embodiment refers to a formulation wherein the one or more phospholipid(s) comprises PC or a mixture of PC with one or more phospholipids selected from the group consisting of phosphatidylinositol (PI), phosphatidylserine (PS), phosphatidylethanolamine (PE), phosphatidylglycerol (PG) and phosphatidic acid (PA) phosphatidylglycerol (PG) or a pharmaceutically acceptable salt of any of the foregoing.

Another preferred embodiment refers to a formulation wherein the one or more phospholipid(s) is PC or a mixture of PC with one or more phospholipids selected from the group consisting of phosphatidylinositol (PI), phosphatidylserine (PS), phosphatidylethanolamine (PE), phosphatidylglycerol (PG) and phosphatidic acid (PA), phosphatidylglycerol (PG) or a pharmaceutically acceptable salt of any of the foregoing.

In one preferred embodiment, a pharmaceutically acceptable salt of a phospholipid is a Na-salt of the phospholipid.

In one preferred embodiment, at least one phospholipid is a PEGylated phospholipid (or pharmaceutically acceptable salt thereof).

In another preferred embodiment, PC or a pharmaceutically acceptable salt thereof is PEGylated.

In yet another preferred embodiment, a formulation comprises PC or a pharmaceutically acceptable salt thereof and PG or a pharmaceutically acceptable salt thereof. In one preferred embodiment, PG is DOPG-Na.

Another more preferred embodiment refers to a formulation wherein the one or more phospholipid(s) comprises PC or a, preferably pharmaceutically acceptable, salt thereof preferably selected from the group consisting of DLPC, DMPC, DPPC, DSPC, POPC, POPC, DEPC, HSPC, HEPC or a, preferably pharmaceutically acceptable, salt of any of the foregoing.

Another more preferred embodiment refers to a formulation wherein the one or more phospholipid(s) comprises a PEGylated PC or a, preferably pharmaceutically acceptable, salt thereof, preferably selected from the group consisting of a PEGylated DLPC, a PEGylated DMPC, a PEGylated DPPC, a PEGylated DSPC, a PEGylated POPC, a PEGylated POPC, a PEGylated DEPC, a PEGylated HSPC, a PEGylated HEPC or a, preferably pharmaceutically acceptable, salt of any of the foregoing.

Another preferred embodiment refers to a formulation, wherein the amount of the one or more phospholipid(s) is between 7%(w/w) and 15%(w/w) compared to the total sum of all components of the liposomal formulation. For example around 11,2% such as (11,2±0,5)%.

Another more preferred embodiment refers to a formulation wherein the one or more phospholipid(s) comprises PC or a, preferably pharmaceutically acceptable, salt thereof. In one even more preferred embodiment, the PC or a, preferably pharmaceutically acceptable, salt thereof is from soybean (e.g. Lipoid S100), egg or synthetic, in a most preferred embodiment, the PC or a, preferably pharmaceutically acceptable, salt thereof, is from soybean.
Another more preferred embodiment refers to a formulation wherein the PC or a, preferably pharmaceutically acceptable, is selected from the group consisting of DLPC, DMPC, DPPC, DSPC, POPC, POPC, DEPC, HSPC, HEPC or combinations thereof.
Another more preferred embodiment refers to a formulation wherein the PG or a, preferably pharmaceutically acceptable, salt thereof is PEGylated and is selected from the group consisting of a PEGylated DLPG, a PEGylated DMPG, a PEGylated DPPG, a PEGylated DSPG, a PEGylated POPG, a PEGylated POPG, a PEGylated DEPG, a PEGylated HSPG, a PEGylated HEPG or a, preferably pharmaceutically acceptable, salt of any of the foregoing.
Another preferred embodiment refers to a formulation wherein the one or more phospholipid(s) comprises PG or a, preferably pharmaceutically acceptable, salt thereof and PG or said salt thereof is a PEGylated PG or a PEGylated salt thereof.
Another more preferred embodiment refers to a formulation wherein the PG or a, preferably pharmaceutically acceptable, salt thereof is selected from the group consisting of DLPG, DMPG, DPPG, DSPG, POPG, POPG, DEPG, HSPG, HEPG.
Another more preferred embodiment refers to a formulation wherein the PG or a, preferably pharmaceutically acceptable, salt thereof is PEGylated and is selected from the group consisting of a PEGylated DLPG, a PEGylated DMPG, a PEGylated DPPG, a PEGylated DSPG, a PEGylated POPG, a PEGylated POPG, a PEGylated DEPG, a PEGylated HSPG, a PEGylated HEPG or a, preferably pharmaceutically acceptable, salt of any of the foregoing.

**(iv) agent(s) (bulking agent and/or tonicity adjusting agent)**

Especially when components of a formulation according to the invention should be lyophilizable or the preparation of a formulation according to the invention via a method according to the invention requires a lyophilization step, the presence of a bulking agent is advantageous. Examples of preferred bulking agents are glycine, arginine, proline, or any other amino acid known to be suitable as a bulking agent or a saccharide component (component (iv) or (f), respectively). Preferred saccharide components are selected from the group consisting of sucrose, trehalose, arabinose, erythritol, fructose, galactose, glucose, lactose, maltitol, maltose, maltotriose, mannitol, mannobiose, mannose, ribose, sorbitol, xylitol, xylose, dextran, or a mixture thereof.
One more preferred embodiment refers to a formulation, wherein (f) in an aqueous phase in a method according to the invention (and, thus, (iv) in a liposomal formulation according to the invention, respectively) is trehalose.
One preferred embodiment refers to a liposomal formulation, wherein one agent is trehalose and wherein the amount of trehalose in the formulation is between 1,5%(w/w) and 3,5%(w/w), such as (2,5$\pm$0,5)%(w/w) based on the total weight of the liposomal formulation.
Another preferred embodiment refers to a pharmaceutical liposomal formulation comprising a tonicity adjusting agent.
Such a tonicity adjusting agent, preferably a pharmaceutically acceptable tonicity adjusting agent, should be present in a pharmaceutical liposomal formulation. It can be added during preparing the liposomal formulation by mixing a lyophilizate according to the invention with an aqueous phase comprising said tonicity adjusting agent or it can be provided during a rehydration step of a lyophilizate.
Alternatively, it can already be present in the lyophilizate in that the tonicity adjusting agent is part of the aqueous phase which is used in a method according to the invention to prepare a lipopeptide according to the invention. The skilled person is aware that a tonicity adjusting agent can also be partially part of such an aqueous phase and partially part of an aqueous phase. The skilled person can easily calculate the total amount of a tonicity adjusting agent which is required to have the right concentration in the final pharmaceutical liposomal formulation to have an isotonic effect in regard of a medicament which is provided to a patient.
The skilled person will recognize that a tonicity adjusting agent is in some cases also a bulking agent, e.g. in case the tonicity adjusting agent is a saccharide component such as glucose or trehalose.
Other tonicity adjusting agents such as NaCl do not have a bulking function. Thus, if using such a "single functional component", e.g., NaCl, a formulation may comprise a bulking agent e) and a tonicity adjusting agent e). It may even comprise a bulking agent (e.g. trehalose) as well as "two" tonicity adjusting agents (trehalose and, e.g., NaCl).
Tonicity adjusting agents are well known to the skilled person. In one preferred embodiment, the tonicity adjusting agent is selected from the group consisting of dextrose, glucose, mannitol, sucrose, lactose, trehalose, glycine, arginine, proline and NaCl, in particular glycine, mannitol, trehalose, glucose and NaCl, even more particularly glycine and NaCl, most preferably NaCl.
The skilled person is aware how to choose the right concentration of a specific tonicity adjusting agent in a

formulation, preferably for subcutaneous injection.

In a preferred embodiment, the tonicity adjusting agents is NaCl and the concentration of NaCl in the liposomal formulation is between 0,8% and 1%, more preferably around 0,9%, e.g. 0,9%±0,1%, more preferably 0,9%, based on the total weight of the pharmaceutical liposomal formulation.

Depending on the tonicity adjusting agent and the kind of the formulation (e.g. a pharmaceutically acceptable liposomal formulation or a lyophilizate), the skilled person is well aware how to calculate the amount of one or more tonicity adjusting agent(s) present in a (final) formulation for administration to a patient with a physiological concentration.

### (v) Dutogliptin

In one preferred embodiment, the concentration of Dutogliptin in a liposomal formulation is between 25 mg/ml and 60 mg/ml, more preferably between 30 mg/ml and 50 mg/ml, e.g. an injection with a volume of 2 ml comprises between 50 mg and 120 mg, more preferably between 60 mg and 100 mg Dutogliptin.

## Lyophilizate

One further aspect of the present invention refers to a lyophilizate comprising Dutogliptin ((g)), (a), optionally (c), optionally (e), and optionally (f) as defined in the methods herein.

Preferably, the total amount of Dutogliptin is between 50 mg and 120 mg, more preferably between 60 mg and 100 mg.

Preferably, wherein

the amount of (g) is between 12%(w/w) and 53%(w/w) based on the total amount of the lyophilizate;

the amount of (a) is between 7,2%(w/w) and 73%(w/w) based on the total amount of the lyophilizate;

the amount of (c) is at most 24,3%(w/w), preferably (c) comprises cholesterol and the amount of cholesterol is between 4,8%(w/w) and 9,7%(w/w) based on the total amount of the lyophilizate;

the amount of (e) is at most 4,9%(w/w) based on the total amount of the lyophilizate;

the amount of (f) is at most 24,3%(w/w), preferably (f) comprises trehalose and the amount of trehalose is between 12%(w/w) and 53%(w/w) based on the total amount of the lyophilizate.

In one preferred embodiment, (c) consists of cholesterol.

In another preferred embodiment, (f) consists of trehalose.

Another aspect refers to the use of a lyophilizate comprising Dutogliptin as described herein for the preparation of a medicament, preferably a pharmaceutical liposomal Dutogliptin formulation.

## Liposome size

In yet another embodiment, the liposomes of a liposomal formulation according to the invention have a D90 size distribution of between 1 μm and 4,5 μm.

## Method of treatment / use

One aspect refers to the use of a liposomal formulation according to the invention for the preparation of a medicament to treat diabetes, in particular diabetes type 2.

## Method of preparation

Another aspect refers to method (A) for the preparation of a liposomal formulation according to the invention.

Another aspect refers to method (B) for the preparation of a liposomal formulation according to the invention.

Another aspect refers to method (C) for the preparation of a liposomal formulation according to the invention.

**Organic phase**

**(a) Phospholipid**

In one preferred embodiment, the one or more phospholipid(s) in an organic phase comprises PC or a mixture of PC with one or more phospholipids selected from the group consisting of phosphatidylinositol (PI), phosphatidylserine (PS), phosphatidylethanolamine (PE), phosphatidylglycerol (PG) and phosphatidic acid (PA) phosphatidylglycerol (PG) or a pharmaceutically acceptable salt of any of the foregoing.

In one preferred embodiment, the one or more phospholipid(s) in an organic phase comprises PC or a pharmaceutically acceptable salt thereof and PG or a pharmaceutically acceptable salt thereof.

In one preferred embodiment, at least one phospholipid is a PEGylated phospholipid.

Another more preferred embodiment refers to a formulation wherein the one or more phospholipid(s) comprises PC or a, preferably pharmaceutically acceptable, salt thereof preferably selected from the group consisting of DLPC, DMPC, DPPC, DSPC, POPC, POPC, DEPC, HSPC, HEPC or a, preferably pharmaceutically acceptable, salt of any of the foregoing.

Another more preferred embodiment refers to a formulation wherein the one or more phospholipid(s) comprises a PEGylated PC or a, preferably pharmaceutically acceptable, salt thereof, preferably selected from the group consisting of a PEGylated DLPC, a PEGylated DMPC, a PEGylated DPPC, a PEGylated DSPC, a PEGylated POPC, a PEGylated POPC, a PEGylated DEPC, a PEGylated HSPC, a PEGylated HEPC or a, preferably pharmaceutically acceptable, salt of any of the foregoing.

Another more preferred embodiment refers to a formulation wherein the one or more phospholipid(s) comprises PC or a, preferably pharmaceutically acceptable, salt thereof. In one even more preferred embodiment, the PC or a, preferably pharmaceutically acceptable, salt thereof is from soybean (e.g. Lipoid S100), egg or synthetic, in a most preferred embodiment, the PC or a, preferably pharmaceutically acceptable, salt thereof, is from soybean.

Thus, one preferred embodiment refers to a method to prepare a liposomal formulation, wherein the organic phase comprises between 3%(w/w) and 30%(w/w) based on the sum of (a), (b) and (c), preferably between 15%(w/w) and 28%(w/w) based on the sum of (a), (b) and (c), such as (22,3$\pm$0,5)%(w/w) based on the sum of (a), (b) and (c).

Another preferred embodiment refers to an organic phase wherein PG or a, preferably pharmaceutically acceptable, salt thereof is a PEGylated PG or a PEGylated, preferably pharmaceutically acceptable, salt thereof.

Another more preferred embodiment refers to an organic phase wherein the PG or a, preferably pharmaceutically acceptable, salt thereof is selected from the group consisting of DLPG, DMPG, DPPG, DSPG, POPG, POPG, DEPG, HSPG, HEPG.

Another more preferred embodiment refers to an organic phase wherein the PG or a, preferably pharmaceutically acceptable, salt thereof is PEGylated and is selected from the group consisting of a PEGylated DLPG, a PEGylated DMPG, a PEGylated DPPG, a PEGylated DSPG, a PEGylated POPG, a PEGylated POPG, a PEGylated DEPG, a PEGylated HSPG, a PEGylated HEPG or a, preferably pharmaceutically acceptable, salt of any of the foregoing.

Another preferred embodiment refers to an organic phase, wherein the one or more phospholipid(s) comprises or consists of PC and the amount of PC is between 3,2%(w/w) and 29,8%(w/w) based on the sum of (a), (b) and (c), preferably between 15,2%(w/w) and 27,8%(w/w) based on the sum of (a), (b) and (c), such as (22,1$\pm$0,5)%(w/w) based on the sum of (a), (b) and (c).

Another preferred embodiment refers to an organic phase, wherein the one or more phospholipid(s) comprises or consists of PC (or a pharmaceutically acceptable salt thereof) and PG (or a pharmaceutically acceptable salt thereof) and the amount of PC (or a pharmaceutically acceptable salt thereof) is between 3,1%(w/w) and 29,9%(w/w) based on the sum of (a), (b) and (c), preferably between 15,1%(w/w) and 27,9%(w/w) based on the sum of (a), (b) and (c), such as (22,1 $\pm$0,5)%(w/w) based on the sum of (a), (b) and (c) and the combined amount of DOPG and PG is between 0,1%(w/w) and 0,3%(w/w) based on the sum of (a), (b) and (c), preferably between 0,15%(w/w) 0,28%(w/w) based on the sum of (a), (b) and (c), such as (0,23$\pm$0,1)%(w/w) based on the sum of (a), (b) and (c).

**(b) Organic solvent**

For the organic phase, appropriate water-miscible lyophilizable organic solvent can be selected by the skilled formulator.

The organic solvent should be miscible with water at standard conditions (25°C and 1,013 bar). Moreover, an organic solvent should be lyophilizable, i.e. the solvent can be removed by sublimation (the transition of a substance directly from the solid to the gas state, without passing through the liquid state). The skilled person is aware how to choose suitable organic solvents as well as suitable temperatures and pressures to lyophilize an organic solvent by, e.g., using a pressure temperature (PT) diagram of a solvent known in the art.

Preferred organic solvents are selected from the group consisting of anisole, ethylacetate, 1,4-dioxane,

dimethylcarbonate, dimethylsulfoxide, glycofurol, N,N-dimethylacetamide, N,N-dimethylformamide, N-methyl-2-pyrrolidone (NMP), isopropylideneglycerol and alcohols such as methanol, ethanol, propanol, butanol or pentanol.

In one preferred embodiment, the organic solvent is anisole (CAS 100-66-3).

In one preferred embodiment, the organic solvent is ethylacetate (CAS 141-78-6).

In one preferred embodiment, the organic solvent is 1,4-dioxane (CAS 123-91-1).

In one preferred embodiment, the organic solvent is dimethylcarbonate (CAS 616-38-6).

In one preferred embodiment, the organic solvent is dimethylsulfoxide (CAS 67-68-5).

In one preferred embodiment, the organic solvent is glycofurol (CAS 31692-85-0).

In one preferred embodiment, the organic solvent is N,N-dimethylacetamide (CAS 127-19-5).

In one preferred embodiment, the organic solvent is N,N-dimethylformamide (CAS 68-12-2).

In one preferred embodiment, the organic solvent is N-methyl-2-pyrrolidone (CAS 872-50-4).

In one preferred embodiment, the organic solvent is isopropylideneglycerol (CAS 100-79-8).

In one preferred embodiment, the organic solvent is 1-butanol (CAS 71-36-3).

In one preferred embodiment, the organic solvent is 2-butanol (CAS 78-92-2).

In one preferred embodiment, the organic solvent is tert-butanol (CAS 75-65-0).

It was surprisingly found that the use of butanol, preferably tert-butanol (TBA) leads to a preferred nano-disperse system when combining the organic phase from step A.1/B.1/C.1, respectively, with the aqueous phase from step A.2/B.2/C.2.

Thus, in one more preferred embodiment, the organic solvent is tert-butanol (CAS 75-65-0).

In one preferred embodiment, the one or more organic solvent(s) is/are present in an organic phase, the amount of the said one or more organic solvent(s) is/are between 60%(w/w) and 97%(w/w) based on the sum of (a), (b) and (c), preferably between 65%(w/w) and 85%(w/w) such as (74,5$\pm$2)%(w/w) based on the sum of (a), (b) and (c) in Method A, based on the sum of (i), (ii) and (iii) in Method B or based on the sum of (1), (2) and (3) in Method C, respectively.

### (c) Further organic components

Generally any organic component may be present. However, it is preferred if an organic component is pharmaceutically acceptable for a human being.

Preferably, organic component are saccharides such as trehalose, polysaccharides such as cellulose or sodium carboxymethylcellulose, lipids such as cholesterol, amino acids, amino acid chains etc..

Cholesterol is known to have an influence on the stability of liposomes and on drug release.

Therefore, in one preferred embodiment, an organic phase of a method according to the invention preferably further comprises cholesterol or a derivative thereof.

Another preferred embodiment refers to said organic phase, wherein the further organic component is selected from the group consisting of cholesterol or sodium cholesteryl sulfate. More preferably, the further organic component is cholesterol.

Another preferred embodiment refers to a formulation, wherein the amount of cholesterol is between 1%(w/w) based on the sum of (a), (b) and (c) and 8%(w/w) based on the sum of (a), (b) and (c), preferably between 2%(w/w) and 4,4%(w/w), such as (3,2$\pm$0,3)%(w/w) based on the sum of (a), (b) and (c).

In one preferred embodiment, the sum of the weight of (a), (b) and (c) is 95%(w/w) or more based on the total weight of the organic phase, preferably 98%(w/w) or more based on the total weight of the organic phase.

In one preferred embodiment, an organic phase consists of (a), (b) and (c).

In another preferred embodiment, (a) consists of PC and PG, preferably PC and DOPG (b) consists of TBA, and (c) consists of cholesterol.

In another preferred embodiment, an organic phase consists of (a), (b) and (c) and (a) comprises PC and PG, preferably PC and DOPG, (b) comprises TBA, and (c) comprises cholesterol.

In another preferred embodiment, an organic phase consists of (a), (b) and (c) and (a) consists of PC and PG, preferably PC and DOPG, (b) consists of TBA, and (c) consists of cholesterol.

## Aqueous phase

### (d) Aqueous medium

The aqueous medium of an aqueous phase is water or a combination of water with a solvent selected from the group consisting of propylene glycol, ethanol, isopropanol, glycerol, glycol ethers (such as monobutyl ether of ethyleneglycol or propyleneglycol, or the monoethylether or the monomethylether of diethyleneglycol or any combination thereof), polyethylenglycol (PEG) 300, PEG 400, in particular propylene glycol and ethanol, wherein the amount of water of the total amount of solvent of the aqueous medium is at least 60%, preferably at least 90%, more preferably at least 95%.

In one preferred embodiment, water is the only solvent in an aqueous medium and, thus, the only solvent of an aqueous phase.

Generally, an aqueous phase comprises an aqueous medium, wherein the amount of the aqueous medium is between 76%(w/w) and 92%(w/w) based on the sum of (d), (e), (f) and (g), more preferably between 80%(w/w) and 88%(w/w) based on the sum of (d), (e), (f) and (g), such as (84.2±2)%(w/w).

**(e) further components selected from the group consisting of a buffer system, and pH adjusting acids and/or bases (e.g. NaOH, HCl)**

Optionally, the aqueous phase may comprise a buffer-system. Suitable pharmaceutically acceptable buffer systems are well-known to the skilled person. Non-limiting examples are systems based on sodium phosphate, citric acid, acetic acid, tromethamine (TRIS),histidine, gluconic, lactic, tartaric, aspartic, glutamic acid, citrate, fumarate, alpha-ketoglutarate, malate, and succinate. The skilled person is aware how to choose a buffer for a specific pH value. For example, acetate buffer (e.g., acetate/acetic acid) can be used for pH-adjustment between 3.7 to 6.5, phosphate buffer or citrate buffer (e.g., $Na_2HPO_4$/citric acid, $Na_2HPO_4$/ $NaH_2PO_4$ or $Na_2HPO_4$/NaOH) can be used for pH adjustment between 5.4 and 8.0, sodium citrate/citric acid can be used for pH adjustment between 3.0 and 6.2.

Optionally, the aqueous phase may comprise HCl and/or NaOH. The two components can, e.g. be used to adjust the pH of an aqueous phase, optionally comprising Dutogliptin, to be in a range is between 6 and 8, preferably between 7 and 8, more preferably between 7.0 and 7.8, such as 7.4±0.2.

**(f) bulking agent**

The aqueous phase may optionally comprise a bulking agent. Bulking agents suitable for a liposomal Dutogliptin formulation and, thus for an aqueous phase to prepare such a formulation are already described above.

In one preferred embodiment, the bulking agent is trehalose.

In another preferred embodiment, a bulking agent is present in an aqueous phase in an amount of between 1 % (w/w) and 1 0%(w/w) based on the sum of (d), (e), (f) and (g), more preferably between 3%(w/w) and 8%(w/w) based on the sum of (d), (e), (f) and (g), such as (5±1)%(w/w) based on the sum of (d), (e), (f) and (g).

In yet another preferred embodiment, a bulking agent is trehalose and the trehalose is present in an aqueous phase in an amount of between 1%(w/w) and 10%(w/w) based on the sum of (d), (e), (f) and (g), more preferably between 3%(w/w) and 8%(w/w) based on the sum of (d), (e), (f) and (g), such as (5±1)%(w/w) based on the sum of (d), (e), (f) and (g).

**(g) Dutogliptin**

Optionally, an aqueous phase comprises Dutogliptin. However, for a method according to the invention, the proviso is that either an aqueous phase or an aqueous solution (for reconstituting a lyophilizate) comprises Dutogliptin.

In one preferred embodiment, an aqueous phase comprises Dutogliptin and Dutogliptin is provided in its free base form.

In yet another preferred embodiment, an aqueous phase comprises Dutogliptin and Dutogliptin is provided in form of one of its fatty acid salts, preferably its (sodium) laurate salt, its (sodium) myristate salt, its (sodium) palmitate salt, its (sodium) stearate salt or its (sodium)cholate hydrate salt.

In one preferred embodiment, the amount of Dutogliptin in an aqueous phase is between 5%(w/w) and 22%(w/w) based on the sum of (d), (e), (f) and (g), preferably between 6%(w/w) and 14%(w/w) based on the sum of (d), (e), (f) and (g), such as (6.6±0,5)%(w/w) based on the sum of (d), (e), (f) and (g) when the Dutogliptin is provided in its free base form; or such as (16.3±3)%(w/w) based on the sum of (d), (e), (f) and (g) when the Dutogliptin is provided in one of its free fatty acid salt forms.

In one preferred embodiment, the method for the preparation of a liposomal formulation is method (A) and the aqueous phase is prepared by providing (g) Dutogliptin in its tartrate salt form.

In an even more preferred embodiment, the method for the preparation of a liposomal formulation is method (A) and the aqueous phase is prepared by providing (g) Dutogliptin in its free base form.

In another preferred embodiment, the method for the preparation of a liposomal formulation is method (B) and the aqueous phase does not comprise (g) and the aqueous solution comprises (g), preferably the aqueous solution is prepared by using Dutogliptin in its tartrate salt form.

In another preferred embodiment, the method for the preparation of a liposomal formulation is method (B) and the aqueous phase does not comprise (g) and the aqueous solution comprises (g), preferably in its tartrate salt form.

In yet another preferred embodiment, the method for the preparation of a liposomal formulation is method (B) and the aqueous phase does not comprise (g) and the aqueous solution comprises (g), preferably the aqueous solution is

prepared by using Dutogliptin in its free base form.

In another preferred embodiment, the method for the preparation of a liposomal formulation is method (B) and the aqueous phase does not comprise (g) and the aqueous solution comprises (g), preferably in its free base form.

In a very preferred embodiment, all components such as an aqueous medium, a buffer and a tonicity adjusting agent are pharmaceutically acceptable components.

Usually, a buffer is present in a concentration between 0.05 mM and 100 mM such as between 1 mM and 50 mM.

In one preferred embodiment, an aqueous phase comprises (d) consisting of water, (e) comprising NaOH and/or HCl (e.g. traces of one or both components for adjusting the pH), (f) comprising, preferably consisting of, trehalose, and (g) consisting of Dutogliptin free base (see, e.g., method A).

In another preferred embodiment, an aqueous phase comprises (d) water, (e) NaOH and/or HCl (e.g. traces of one or both components for adjusting the pH), (f) trehalose, and (g) Dutogliptin in one of its fatty acid salt forms (see, e.g., method C).

In yet another preferred embodiment, an aqueous phase comprises (d) water, (e) NaOH and/or HCl (e.g. traces of one or both components for adjusting the pH), (f) trehalose, and (g) Dutogliptin free base (see, e.g., method A). Preferably, the amount of (d) is between 80%(w/w) and 91%(w/w) based on the sum of (d), (e), (f) and (g), the amount of (e) is less than 0.1%(w/w) based on the sum of (d), (e), (f) and (g), the amount of (f) is between 3%(w/w) and 8%(w/w) based on the sum of (d), (e), (f) and (g), and the amount of (g) is between 6%(w/w) and 14%(w/w) based on the sum of (d), (e), (f) and (g).

In another preferred embodiment, an aqueous phase comprises (d) water, (e) NaOH and/or HCl (e.g. traces of one or both components for adjusting the pH), (f) trehalose, and (g) Dutogliptin in one of its fatty acid salt forms (see, e.g., method C). Preferably, the amount of (d) is between 80%(w/w) and 91%(w/w) based on the sum of (d), (e), (f) and (g), the amount of (e) is less than 0,1%(w/w) based on the sum of (d), (e), (f) and (g), the amount of (f) is between 3%(w/w) and 8% (w/w) based on the sum of (d), (e), (f) and (g), and the amount of (g) is between 6%(w/w) and 14%(w/w) based on the sum of (d), (e), (f) and (g).

In another preferred embodiment, the sum of the weight of (d), (e), (f) and (g) is 95%(w/w) or more based on the total weight of the aqueous phase, preferably 98%(w/w) or more based on the total weight of the aqueous phase.

In one preferred embodiment, an aqueous phase consists of (d), (e), (f) and (g).

**Aqueous solution**

An aqueous solution as used herein comprises a (pharmaceutical) aqueous medium, preferably water or a combination of water with any of the solvents selected from the group consisting of propylene glycol, ethanol, isopropanol, glycerol, glycol ethers (such as monobutyl ether of ethylenglycol or propyleneglycol, or the monoethylether or the monomethylether of diethyleneglycol or any combination thereof), polyethylenglycol (PEG) 300, PEG 400, in particular propylene glycol and ethanol or a combination thereof, preferably wherein the amount of water of the total amount of solvent is at least 80%. In an even more preferred embodiment, water is the only solvent of an aqueous solution.

In one embodiment, an aqueous solution consists of water or a combination of water with any of the solvents selected from the group consisting of propylene glycol, ethanol, isopropanol, glycerol, glycol ethers (such as monobutyl ether of ethylenglycol or propyleneglycol, or the monoethylether or the monomethylether of diethyleneglycol or any combination thereof), polyethylenglycol (PEG) 300, PEG 400, in particular propylene glycol and ethanol or a combination thereof, wherein the amount of water of the total amount of solvent is at least 80%. In an even more preferred embodiment, a pharmaceutical aqueous solution consists of water.

A further preferred embodiment refers to an aqueous solution comprising a solvent as described herein and a tonicity adjusting agent. Preferred tonicity adjusting agents for use in an aqueous solution are already described for the formulations according to the invention.

A further preferred embodiment refers to an aqueous solution comprising a solvent as described herein and a tonicity adjusting agent and a buffer. Preferred tonicity adjusting agents and buffer systems for use in an aqueous solution are already described for the liposomal formulations according to the invention. A preferred tonicity adjusting agent is NaCl, preferably the amount of NaCl in the aqueous solution is $(0.9 \pm 0.1)$%(w/w), most preferably 0.9%(w/w) based on the total amount of the aqueous solution.

Preferably, the pH of a pharmaceutical aqueous solution is chosen so that the pH of the resulting liposomal formulation is between 6 and 8, preferably between 7 and 8.

In one embodiment, an aqueous formulation also comprises Dutogliptin, preferably in form of its tartrate salt form or its free base form, more preferably in form of its tartrate salt form. Preferably, the amount of Dutogliptin is between 5% (w/w) and 13%(w/w), more preferably $(10 \pm 0.5)$%(w/w) based on the total weight of the aqueous solution when the Dutogliptin is present in its tartrate salt form; or $(6 \pm 0.4)$%(w/w) based on the total weight of the aqueous solution when the Dutogliptin is present in its free base form.

In one embodiment, a method for the preparation of a liposomal Dutogliptin formulation is method (B) and the aqueous solution comprises Dutogliptin, preferably in its tartrate salt form.

In another preferred embodiment, a method for the preparation of a liposomal Dutogliptin formulation is method (B) and the aqueous solution comprises Dutogliptin, preferably in its free base form.

The skilled person is aware that, depending on the preparation method according to the invention, a lyophilizate can already comprise a buffer system or such a buffer system is provided with a pharmaceutical aqueous solution. The skilled person can calculate the requirements on a pharmaceutical aqueous solution for preparing the final liposomal formulation without undue burden.

Preferred tonicity adjusting agents, buffer, solvent, phospholipid, phosphatidylglycerol, lipopeptide, Cholesterol (and derivatives thereof), saccharose components, ratios and amounts of any of the foregoing which can be used in a method of the invention are already described above for the formulations according to the invention.

**Calculation of concentrations/amounts of organic phase, aqueous phase and aqueous solution**

The skilled person is able to calculate the concentrations and amounts of the various components for the organic phase and aqueous phase to be combined in step A.3/B.3/C.3 and the concentrations and amounts of the various components of the aqueous solution of step to arrive at a formulation according to the invention with the requirements regarding the ratios between the various components, without undue burden.

Surprisingly it was found the formulation of step A.3/B.3/C.3 is a nano-dispersed system, wherein the D90 of the lipophilic vesicles is 60 nm or less, preferably less than 25 nm, more preferably 20 nm or less, such as between 10 nm and 20 nm e.g., around 15 nm, or between 3 nm and 10 nm, e.g., around 5 nm.

In other words, being bound to the explanation, by combining the organic phase from A.1/B.1/C.1 and the aqueous phase from A.2/B.2/C.2 no liposomes are resulting but a nano-disperse system with a D90 of the particle size of 60 nm or less, preferably less than 25 nm, presumably micelles. This nano-dispersion can be sterile filtered.

In one preferred embodiment, the ratio between the organic phase and the aqueous phase is between 3:1 (v/v) and 1:3(v/v), even more preferably between 2:1 (v/v) and 1:2 (v/v) such as around 1:1 (v/v). For explanation's sake, "(v/v)" refers to volume ratios of the two phases, e.g., a ratio of 1:1 refers to 1ml of an organic phase and 1 ml of an aqueous phase.

In one preferred embodiment, the D90 of the vesicles in a resulting nano-dispersed system, when the ratio between the two phases is 1:1, is 15 nm or less, more preferably 10 nm or less such as between 3 nm and 10 nm. Without being bound to the explanation, the high ratio of organic solvent may lead to a predominantly molecular solution of the components within the solvent mixture.

In another preferred embodiment, the D90 of the vesicles in a nano-dispersed system, when the ratio between the two phases is 1:3, is 60 nm or less, more preferably less than 25 nm, even more preferably 20 nm or less such as between 5 nm and 20 nm. Without being bound to the explanation, at this higher proportion of water a micellar solution is formed showing the typical size and homogeneity of micelles. The mixture shows a nearly clear appearance with slight Tyndall effect and is freely filterable through a sterile filter with 0.22 μm nominal pore size.

At a solvent mixture ratio of 1:3 the resulting preparation is turbid. Size distribution measurement by DLS reveals a broad inhomogeneous spectrum of vesicles having a mean size of about 1.000 μm (1000nm). This dispersion can be filtered through a membrane sterile filter with 0.22 μm nominal pore size only by applying high pressure.

Usually, all steps of method (A), (B) and (C) can be performed around and at room temperature (25 °C) and around and at standard pressure (101.325 kPa). If not stated otherwise, e.g. for the lyophilization step, steps can individually be performed at temperatures preferably between 0 °C and 40 °C, more preferably between 15 °C and 35°C such as between 18 °C and 28 °C. Although any of the steps can be individually carried out also at higher and lower pressures, preferably any of the steps is individually carried out at a pressure between 90 kPa and 112 kPa, more preferably between 95 kPa and 116 kPa, most preferably around standard pressure, e.g., 101.325 kPa + 2%.

The skilled person is well-aware of freeze drying (lyophilization) techniques. Usually, freeze drying of a formulation according to the invention is performed at a temperature between + 40°C and -40°C, preferably between + 30°C and -10 °C. A freeze-drying step may be repeated one or more times. Usually, the pressure is between 1000 hPa and 0.001 hPa (1hPa = 1 mbar). Preferably, a freeze dying step is performed at a pressure between 1 hPa and 0.01 hPa such as around 0.1 hPa.

Optionally, the method comprises one or more further steps.

Preferably one further step in a method according to the invention is the step "sterile filtering" a nano-dispersed system or a reconstituted lyophilizate solution.

Preferably, the sterile filtering is done by using a membrane filter, e.g. a PVDF membrane filter. Preferably, the nominal pore size of a membrane filter is 200 nm or less.

It should be noted, for homogenization purpose, it is sufficient to gently swirl or vortex the obtained liposomal dispersion. It is not required to reduce the size of the liposomes by further, complicated steps, when the liposomal formulation should be suitable for injection into a patient in need of a lipopeptide formulation.

Preparation of a liposomal Dutogliptin formulations can be carried out in accordance with or similar to the Examples described below.

Preparation of a liposomal Dutogliptin formulation using a Dutogliptin fatty acid salt can be carried out similar to the Example 1 by substituting the Dutogliptin tartrate salt by a Dutogliptin fatty acid salt.

Preparation of a liposomal Dutogliptin formulation using a Dutogliptin fatty acid salt can be carried out similar to Example 2 by substituting the Dutogliptin tartrate salt by a Dutogliptin fatty acid salt.

Preparation of a liposomal Dutogliptin formulation using a Dutogliptin fatty acid salt can be carried out by adding a Dutogliptin fatty acid salt to the organic phase and then following the route of Example 1 but omitting the Dutogliptin in the aqueous phase.

Preparation of a liposomal Dutogliptin formulation using the Dutogliptin free base form can be carried out similar to the Example 1 by substituting the Dutogliptin tartrate salt by the Dutogliptin free base form.

Preparation of a liposomal Dutogliptin formulation using the Dutogliptin free base form can be carried out similar to Example 2 by substituting the Dutogliptin tartrate salt by the Dutogliptin free base form.

Preparation of a liposomal Dutogliptin formulation using the Dutogliptin free base form can be carried out by adding the Dutogliptin free base form to the organic phase and then following the route of Example 1 but omitting the Dutogliptin in the aqueous phase.

## Kit

A further aspect of the invention refers to a kit comprising a lyophilizate as described herein and separated therefrom an aqueous formulation as described herein to prepare a liposomal formulation according to the invention. For example, the formulation according to the invention is in one container while the pharmaceutical aqueous solution is in another container.

In one preferred embodiment, the lyophilizate comprises Dutogliptin in a total amount of between 60 mg and 100 mg and the amount of Dutogliptin in the lyophilizate is between 12%(w/w) and 53%(w/w) based on the total amount of the lyophilizate; the amount of (a) is between 7.2%(w/w) and 73%(w/w) based on the total amount of the lyophilizate; the amount of (c), if present, is at most 24.3%(w/w), preferably (c) comprises cholesterol and the amount of cholesterol is between 4.8%(w/w) and 9.7%(w/w) based on the total amount of the lyophilizate; the amount of (e), if present, is at most 4.9%(w/w) based on the total amount of the lyophilizate; the amount of (f), if present, is at most 24.3%(w/w), preferably (f) comprises trehalose and the amount of trehalose is between 12%(w/w) and 53%(w/w) based on the total amount of the lyophilizate.

In one preferred embodiment, the pharmaceutical aqueous solution is portioned in another container and the weight of the portion of the pharmaceutical aqueous solution is calculated so that the amount of the sum of and the ratios between the various components i), ii, iii), iv) and v) of the resulting liposomal formulation are those of the liposomal formulation according to the invention after adding the portion of the pharmaceutical aqueous solution to the lyophilizate (or *vice versa*).

In one preferred embodiment, the volume of the aqueous solution to be added to the lyophilizate is between 1.8 ml and 2.2 ml, such as 2 ml. Preferably, the volume of an aqueous solution in a container refers to the volume of a subcutaneous injection.

The mixing of the two components pharmaceutical aqueous solution and lyophilized formulation allows to prepare the pharmaceutical liposomal formulation shortly before administration to a patient.

While the described invention has been described with reference to the specific embodiments thereof it should be understood by those skilled in the art that various changes may be made, and equivalents may be substituted without departing from the true scope of the invention. In addition, many modifications may be made to adopt a particular situation, material, composition of matter, process, process step or steps, to the objective scope of the invention. All such modifications are intended to be within the scope of the claims appended hereto.

Notably, all preferred components mentioned in any of the phases, solutions, formulations, lyophilizates, kits or methods also refer to any of the other phases, solutions, formulations, kits or methods disclosed herein even if not explicitly mentioned therefore and if applicable. For example, when a preferred bulking agent is present in an organic phase, the same preferred agent will be also a preferred bulking agent for a lyophilizate and the resulting liposomal formulation, of course.

## Figures

Figure 1 shows two-phase systems of Dutogliptin aqueous phase (lower phase) and organic TBA phase (upper phase). Dutogliptin aqueous phase (lower phase) + TBA-phase is shown on the left and Dutogliptin aqueous phase (lower phase) + TBA/PC/PG-phase is shown on the right (each 1:1 w/w).

Figure 2 shows the online process data chart. The lyophilization process was completed successfully and the conducted process matched the preset parameters as described in section E5. The following parameters are shown in Figure 2:

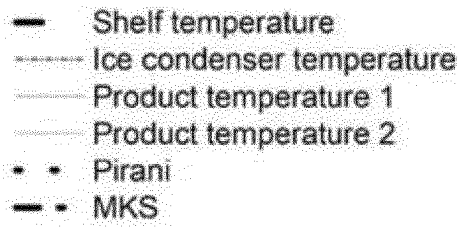


Figure 3 shows a photo of 50 mg Dutogliptin formulation, magnification: 40x (A/4 polarization microscope). Various types of liposomes are present: small monolamellar vesicles, large monolamellar vesicles, multilamellar vesicles and oligolamellar vesicles.

## Examples

### E1 Substances and Materials

The following substances and materials were used (Table ).

**Table 1: Substances and materials**

| Substances | Lot-no.: | Manuf. / Suppl. | Order-no.: |
|---|---|---|---|
| Dutogliptin injection drug product (DP),100 mg/mL (free base) | B170433 (DUTO03 inverted) | - / GBA Pharma labs | - |
| Lipoid S100 (PC) | - | Lipoid | 579000-1190738-13/703 |
| DOPG-Na 18:1/18:1 (PG) | - | Lipoid | 564300-2210011-01/003 |
| tert-butanol (TBA) | 4323.1 | Carl Roth | 081167163 |
| cholesterol | 8866.1 | Carl Roth | 371176656 |
| SDS ultra pure, ≥ 99.5 % | 141168156 | Carl Roth GmbH | 2326 |
| Acetonitrile (ACN), Chromasolv™, gradient grade, ≥ 99.9 % | K1550 | Honeywell | 34851 |
| Trifluoroacetic acid (TFA), ≥ 99.9 %, for peptide synthesis | 429289720 | Carl Roth GmbH | P088.1 |
| Demineralised water, < 0.2 µS/cm, TOC < 10 ppb | - | ProJect Pharmaceutics | - |
| Saline | 210859141 | B.Braun | 672617400.00 |
| Sodium Laurate | BCCG6618 | Sigma-Aldrich | L9755-5G |
| Sodium Myristate | MKCP8877 | Sigma-Aldrich | M8005-10G |
| Sodium Palmitate | SLCG3426 | Sigma-Aldrich | P9767-5G |
| Sodium Stearate | SLCF4857 | Sigma-Aldrich | S3381-5G |
| Sodium cholate hydrate | SLBH5378V | Sigma-Aldrich | C9282 |
| MOPS, PUFFERAN, ≥99.5% | 460302120 | Carl Roth GmbH | 6979 |
| Calcein | SHBM7472 | Sigma Aldrich | C0875-5G |
| Cobalt(III) chloride | 102462805 | Sigma Aldrich | 60818-50G |

(continued)

| Materials | | | |
|---|---|---|---|
| Waters XTerra MS C18, 3.5 μm, 4.6 x 150 mm | 0240312181 | Waters | 186000440 |
| Omnifix 3 mL syringe | 21H30C8 | B.Braun | 4617022V |
| Stericup 0.22 μm Durapore membrane filter | MP183502G2 | Merck Millipore | S2GVU02RE |
| 96w BRAND plates pureGrade, black | 525276 | BRAND | 781611 |

The following equipment and devices were used:
The following equipment and devices were used:
HPLC Equipment Sys4:

| | |
|---|---|
| Manufacturer: | Agilent Technologies (Santa Clara, California USA) |
| Type: | 1260 Prime Infinity II |
| Flexible Pump | G7104C |
| Thermostated Vialsampler | G7129C |
| Multi Column Thermostat | G7116A |
| DAD HS | G7117C |
| Cell | G4212-60008 1cm, 1 μL |

Pilot freeze dryer (GT2):

| | |
|---|---|
| Manufacturer: | Hof Sonderaniagenbau (Lohra, Germany) |
| 0.36 $m^2$ shelf area | |
| In-vial temperature recording | |
| Differential pressure measurement | |

| | |
|---|---|
| Vacuum regulation: | MKS and Pirani |

Microplate reader

| | |
|---|---|
| Manufacturer: | Tecan (Männedorf, Switzerland) |
| Type: | Safire |
| SN: | 12901300247 |

Additional laboratory equipment

- Magnetic stirrer (IKA Werke)
- Volumetric pipettes (Gilson)
- Camera Equipment (Canon, EOS 600D with DGMacro 105mm 1:2.8)
- pH-meter (Mettler Toledo, SevenMulti with InLab Micro electrode)
- Balance (Kern, EW6200-2NM, ABJ-NM ABS3204N
- Purified water supply (Siemens, Ultra Clean UV UF TM)
- Vortex mixer (Scientific Industries Inc., Vortex Genie II)
- Centrifuge: ThermoScientific, Heraeus Pico 17
- Orbital shaker: Wisd Laboratory Instruments, WiseShake SHO-1D

The following protocol was used for RP-HPLC analysis

**E2 RP-HPLC method**

The intended use of this method is to determine the content and purity of Dutogliptin drug product. The method AM-C16031637-G-01.03 was used as provided. The following chromatographic conditions were used:

| | |
|---|---|
| Column | Waters XTerra MS C18, 3.5 μm, 4.6 x 150 mm |
| Mobile phase A | 25 mM SDS:Acetonitrile: TFA = 600:400:1 (v/v/v) |
| Mobile phase B | 25 mM SDS:Acetonitrile: TFA = 400:600:1 (v/v/v) |
| Flow gradient | |

| Time [min] | % B |
|---|---|
| 0.0 | 0 |
| 25.0 | 0 |
| 45.0 | 100 |
| 60.0 | 100 |
| 60.1 | 0 |
| 70.0 | 0 |

| | |
|---|---|
| Flow rate | 1.0 mL/min |
| Column temperature | 60 °C |
| Injection volume | 60 μL of 1 mg/mL solution |
| Autosampler temperature | 8 °C |
| UV detection | 210 nm; BW: 4 nm; Ref: Off |

<u>25mM Sodium Dodecyl Sulphate (SDS)</u>

Weigh 7.3 g of SDS into 1000 mL of purified water, dissolve and mix well.

Mobile phase A: 25 mM SDS:ACN: TFA = 600:400:1 (v/v/v)

For the preparation of 1L of mobile phase, mix 600 ml of 25 mM fo SDS solution with 400 ml of ACN and 1.0 ml of TFA into a suitable container and mix well. Filter through a 0.45 μm nylon membrane (or other suitable solvent resistant filter) and degas before use

Mobile phase B: 25 mM SDS:ACN: TFA = 400:600:1 (v/v/v)

For the preparation of 1L of mobile phase, mix 400 ml of 25 mM of SDS solution with 600 ml of ACN and 1.0 ml of TFA into a suitable container and mix well. Filter through a 0.45 μm nylon membrane (or other suitable solvent resinstant filter) and degas before use.

Diluent

Mobile phase A

**E3 Salt precipitation / anion exchange**

Assuming that the ion lattice of Dutogliptin tartrate (dissolved in water) is pulled apart so that the individual Dutogliptin and tartrate ions are in solution, an exchange should take place by adding excess fatty acid anions. The resulting hydrophobic salt should form and precipitate due to reduced solubility.

To promote the exchange, the salts, which were dissolved at room temperature, were cooled to 2-8 °C after the addition of Dutogliptin tartrate and stirred overnight.

**E4 Encapsulation efficiency / trapped volume of liposomes**

Liposomes were prepared in a solution of the fluorescent dye, calcein. The fraction of the total volume that was within the liposomes was obtained as the fraction of the fluorescence that remained after adding cobalt(II) ions which, when chaleted by calcein, quench its fluorescence. Multilamellar placebo liposome lyophilizates were prepared according to the method described in Example 1 without Dutogliptin tartrate and reconstituted in 1 mL of Mops (4-morpholinepropanesulfonic acid)-buffered saline (pH 7.2) containing 50 nmol calcein.

6.25 μL liposomal suspension were diluted to 250 μL with Mops-buffered saline, transferred to a 96-well plate and the fluorescence of the suspension was measured before ($F_{tot}$) and after addition of 1.25 μL of 10 mM $CoCl_2$ ($F_{in}$). The fluorescence plate reader parameters are shown in Table 2. Subsequently, 12.5 μL of 10 % Triton X-100 was added and the fluorescence measured again ($F_{totq}$). The latter addition destroyed the integrity of the liposomes. The trapping volume can be calculated with:

$$Trapping\ volume\ (\%\ total) = \frac{F_{in} - (F_{totq} * r)}{F_{tot} - (F_{totq} * r)} * 100$$

with

$F_{tot}$ represents the fluorescence of all the calcein present

$F_{in}$ fluorescence from the internal compartment plus the unquenched fraction of the external compartment

$F_{totq}$ represents the fluorescence of the equilibrium concentration of free calcein

$r$ dilution factor due to Triton X-100 and cobalt addition, present case 1.06

**Table 2 Tecan fluorescence platereader parameters**

| Monochrometer interference filter | | Band width |
|---|---|---|
| Excitation | 490 nm | 5 nm |
| Emission | 520 nm | 5 nm |

**E5 Lyophilization**

Lyo-solution (nano-dispersed system) was filled in sterilized glass vials (1.5 g in 6R vial). Filled vials were stoppered in lyophilization position loaded into the freeze drier. The following lyophilization cycle was performed.

**Table 3: Program of the conservative generic lyophilization process**

| Step | | Shelf temperature | Ice condenser temperature | Pressure (MKS) | Time step | Cumulative time |
|---|---|---|---|---|---|---|
| # | Description | [°C] | [°C] | [mbar] | [h:min] | [h:min] |
| 1 | Loading | 20 | - | 1000 | 00:01 | 00:01 |
| 2 | Freezing Ramp | -45 | - | 1000 | 01:00 | 01:01 |
| 3 | Freezing | -45 | - | 1000 | 03:00 | 03:01 |
| 4 | Vacuum Adjustment | -45 | -70 | 0.05 | 00:30 | 03:31 |
| 5 | Primary Drying Ramp | 0 | -70 | 0.05 | 03:00 | 06:31 |
| 6 | Primary Drying | 0 | -70 | 0.05 | 16:30 | 23:01 |
| 7 | Secondary Drying Ramp | 35 | -70 | 0.05 | 01:00 | 24:01 |
| 8 | Secondary Drying | 35 | -70 | 0.05 | 04:00 | 28:01 |

**E6 Polarization microscopy**

One drop of the well mixed reconstituted liposomal formulation was placed on a microscope slide and covered with a microscope cover glass. Samples were examined at 400x magnification with and without polarizer to identify encapsulated Dutogliptin / liposomes. Photographs are taken at 40x magnification.

The following apparatus were used: Microscope: Axio Lab.A1-HAL with polarizer, Zeiss; Camera Equipment: EOS 600D, Canon

Comparative **Example 1: Method (A) with tartrate salt:**

**Preparation of nano-dispersed system with Dutogliptin tartrate in the aqueous phase**

For the formation of liposomes two phases were required: an organic and an aqueous solution.

Organic phase

For the organic phase phosphatidyl glycerol (PG) and S100 (highly purified soy lecithin) were dissolved in tert-butanol (TBA) by heating (70 °C) and agitation for 40 - 80 min.

The organic phase was cooled down to ambient temperature, as soon as the ingredients were dissolved homogenously.

Aqueous phase

For the aqueous phase, Dutogliptin tartrate solution from Recardio (which was is adjusted to pH 7.4) and Trehalose were added to water and dissolved under agitation.

The aqueous solution is added in small portions to the organic phase under continuous stirring. Depending on the amount of aqueous phase added to the organic phase, different liquid crystal phases may occur. First portions of aqueous solution are dissolved to a clear micellar system. On further addition of aqueous solution cubic, hexagonal and finally lamellar phases with different viscosity and turbidity may emerge. After complete addition of the aqueous phase an opalescent nano-disperse system of low viscosity results.

The final bulk solution is sterile filtered through PVDF membrane filter of 200 nm nominal pore size.

| **Organic phase** | | |
|---|---|---|
| Lipoid S100 | 3.32 | [g] |

(continued)

| Organic phase | | | |
|---|---|---|---|
| DOPG-Na 18:1/18:1 | | 0.035 | [g] |
| Cholesterol | | 0.48 | [g] |
| TBA | | 11.165 | [g] |
| | sum organic phase | 15.0 | g |
| | density | 0.784 | g/mL |
| | Volume | 19.12 | mL |

| Aqueous phase | | | |
|---|---|---|---|
| Dutogliptin Tartrate | | 1.61 | [g] |
| Trehalose | | 0.75 | [g] |
| Water | | 12.64 | [g] |
| | sum aqueous phase | 15.0 | g |
| | density | 1.044 | g/mL |
| | Volume | 14.37 | mL |
| | pH | 7.30 | |

**Direct Encapsulation of Dutogliptin Tartrate**

Typically stable, homogeneous nano-disperse mesophases form in this mixture with the lipids. However, in this case with Dutogliptin tartrate dissolved in the water phase the TBA containing organic and aqueous phase do not form a stable solution and phase separation of the nano-dispersed system occurs after a short time (≤ 5 min.).

A comparison with a lipid-free TBA solution and an aqueous phase with dissolved Dutogliptin tartrate showed that the development of the two-phase system was even more pronounced and formed spontaneous (see Figure 1).

To suppress phase separation the emulsion was constantly stirred during filling. The emulsion was filled in 6R type 1 glass vials at 1.5 g/vial. Filled vials were transferred into the freeze drier and the lyophilization process was started. The lyophilization process was conducted as described in the section E5.

The reconstitution of the lyophilizate cakes for the preparation of the liposomal suspension was carried out by adding water. A stable liposomal suspension quickly formed.

The reconstituted lyophilizate was viewed under a polarization microscope directly after reconstitution/homogenization at 22 °C. Photographs were taken for documentation (see Figure 3).

The liposomal encapsulation of Dutogliptin was studied by determining the amount of free (unencapsulated) Dutogliptin. An equal intra-liposomal and extra-liposomal volume would refer to a 1:1 equilibrium concentration of a hydrophilic active pharmaceutical ingredient (API). A reduction of the Dutogliptin tartrates in the extra-liposomal matrix would result in a positive liposomal encapsulation efficiency.

After reconstitution the liposome dispersion was sucked into a standard single-use 3 mL PP syringe. A 27G x 1" canula (Sterican, B.Braun) was attached and remaining air was removed from the syringe. The content of the syringe was dispensed manually. Evaluation of the push-out force showed that a force of at most 20 N, more preferably of at most 15 N, is sufficient to dispense the liposomal Dutogliptin tartrate formulation

Centrifugation to separate liposomes and aqueous phase is only possible with MLV dispersion. Thus, the amount of Dutogliptin in the extra-liposomal volume was determined by centrifugation of lyophilizates reconstituted by water. The lyophilizates were mixed with 1 mL saline, shaken and stored for 20 minutes at room temperature with gentle stirring to form the liposomes. In the subsequent centrifugation (5 min at 17000xg), the liposomes could not be separated successfully. Successful separation was only achieved after further dilution with 1 mL water (total water volume 2 mL). In order to avoid a possible dilution error, comparison variants were created which were directly mixed with 2 mL saline. The lipid free supernatant of those samples was analyzed by RP-HPLC (Table 4).

Adjusting an equal intra-liposomal and extra-liposomal volume, a 1:1 equilibrium concentration of the hydrophilic Dutogliptin is expected. A reduced concentration of Dutogliptin in the extra-liposomal matrix indicates a positive liposomal encapsulation efficiency.

**Table 4 RP-HPLC results: concentration of Dutogliptin in extra-liposomal volume after liposomal preparation**

| Lyophilizate variant | reconstitution solution | volume reconstitution [mL] | theoretical Dutogliptin conc. [mg/mL] | Dutogliptin conc. RP-HPLC [mg/mL] | encapsulation efficiency** |
|---|---|---|---|---|---|
| 50 mg/vial | WFI | 2 mL | 25 | 27.4 | + 10 % |
| 100 mg/vial | WFI | 4 mL | 25 | 22.9 | -9 % |
| 100 mg/vial | WFI | 2 + 2 mL* | 25 | 20.1 | - 20 % |
| * Lyophilizate was reconstituted with 2 mL and after liposome development further diluted with 2 mL WFI<br>** decrease of extra-liposomal Dutogliptin concentration | | | | | |

**Example 2: Method (B) reconstitution of (placebo) lipid lyophilizate with Dutogliptin aqueous solution and determination of free drug concentration**

Filling of unstable emulsions which tend to phase separation is difficult in large scale manufacturing. Therefore, an alternative manufacturing method was tested:
First, (placebo) liposome lyophilizate were prepared according to the method described above without Dutogliptin tartrate. Then 0.9% NaCl (saline) was added to a Dutogliptin tartrate solution to increase iconicity and zeta-potential. The resulting solution (100 mg/mL, 0.9% NaCl) was added to a (placebo) lyophilizate. A homogenous Dutogliptin liposome emulsion was formed after a few minutes gentle swirling of the vial.

Reconstituted vials were centrifuged directly and after addition of 1 mL of saline (0.9% NaCl solution) (Table 5). The concentration Dutogliptin was determined by means of RP-HPLC within the separated aqueous phase.

Table 5 RP-HPLC results: concentration of Dutogliptin in extra-liposomal volume after liposomal preparation with Dutogliptin tartrate solution (100 mg/mL, 0.9 % NaCl)

| Lyophilizate variant | reconstitution solution | volume reconstitution [mL] | theoretical Dutogliptin conc. [mg/mL] | Dutogliptin conc. RP-HPLC [mg/mL] |
|---|---|---|---|---|
| 100 mg/vial | 100 mg/mL Dutogliptin tratrate, 0.9 % NaCl | 1 mL | 100 | 102.1 |
| 100 mg/vial | 100 mg/mL Dutogliptin tartrate, 0.9 % NaCl / saline | 1 +1 mL* | 50 | 44.35 |
| * Lyophilizate was reconstituted with 1 mL and after liposome development further diluted with 1 mL saline | | | | |

Phase separation was possible by centrifugation. However, due to extremely high g-force during centrifugation liposomes might be damaged and leaked resulting in non-valid results for free drug concentration. However, the results are close to theoretic values. As Dutogliptin tartrate is highly water soluble one would expect that concentration of the drug inside and outside the liposomes is equal and that no drug is associated with the lipid-phase of the vesicles.

To determine the encapsulated amount of drug the volume of water phase trapped by liposomes has to be quantified.

**Trapped volume and derived encapsulation efficacy**

The literature method to determine the liposomal inclusion volume described in 3.6. is subject to some uncertainties. The evaluation of the fluorescence measurements showed increased fluorescence quenching of the liposome solutions compared to free calcein solutions. These are not structurally determined, but are determined by components of the liposomal preparation, which makes an individual consideration of the results dependent on the liposomal composition necessary.

The experiments to determine the liposomal inclusion volume were carried out in triplicate. A series of tests with double-diluted/reconstituted liposome solutions was carried out. An overview is provided in table 6

**Table 6 Normalized fluorescence signals (arbitrary units)**

| | Liposomal preparation 12.8 % (w/w) | | | 2x volume liposomal preparation 7.7 % (w/w) (verification) | | |
|---|---|---|---|---|---|---|
| $F_{tot}$ * | 0.90 | 0.95 | 1.00 | 1.00 | 1.00 | 1.15 |
| $F_{in}$ * | 0.45 | 0.40 | 0.45 | 0.30 | 0.25 | 0.30 |
| $F_{totq}$ * | 0.20 | 0.20 | 0.20 | 0.15 | 0.10 | 0.15 |
| **Trapping vol. (% total)** | 35% | 26 % | 30 % | 17 % | 16 % | 14 % |
| Average | **30 % ± 4 %** | | | **16 % ± 1 %** | | |
| * $F_{tot}$: represents the fluorescence of all the calcein present; $F_{in}$: fluorescence from the internal compartment plus the unquenched fraction of the external compartment; $F_{totq}$: represents the fluorescence of the equilibrium concentration of free calcein | | | | | | |

With a mass-dependent liposomal proportion of 12.8% (w/w) of the reconstituted lyophilisate, an inclusion volume of about 30% of total volume was be determined.

The encapsulation efficacy for a reconstituted liposomal formulation having 12.8% lipids, 100 mg total drug and 1.0 mL saline was calculated by the equation:

$$m_L = 100\ mg - 100\ mg/mL + V_L * 0.3\ mL*100mg/mL = 30\ mg$$

$m_L$ = liposomal encapsulated mass of DGT
$V_L$ = water phase volume of liposomes

Conclusion: About 1/3 of total drug is encapsulated within vesicles.

Comparative

**Example 3: Method (C): Preparation of liposomal Dutogliptin formulation with Dutogliptin fatty acid salts**

Five different physiologic fatty acids (as sodium salts) were prepared by anion exchange of the tartrates with a double molar addition of the salts at a reduced temperature (reduced Dutogliptin tartrate solubility). The most promising fatty acids were selected for further preparation. The solubility of the fatty acid salts at 2-8 °C was examined (see Table ).

**Table 7 Fatty acid sodium salts for preparation of water insoluble Dutogliptin salts**

| Substance | Amount of substance [mol] | Solubility 25 °C | Solubility 2-8 °C | Precipitation after addition of Dutogliptin tartrate solution |
|---|---|---|---|---|
| Dutogliptin tartrate | 0.41 mmol | 1 mL | 1mL | - |
| Sodium Laurate | 0.83 mmol | 10 mL | not soluble | n.a. |
| Sodium Myristate | 0.83 mmol | > 10 mL | not soluble | n.a. |
| Sodium Palmitate | 0.83 mmol | > 10 mL | not soluble | n.a. |
| Sodium Stearate | 0.83 mmol | > 10 mL | not soluble | n.a. |
| Sodium cholate hydrate | 0.83 mmol | 5 mL | 5 mL, clear solution | no precipitation |

The tests on the solubility of the sodium salts showed that of the salts used, only the sodium cholate remained sufficient soluble under the given conditions (cooling down to 2-8 °C). After the addition of 100 mg/ml Dutogliptin tartrate solution, the solutions became slightly yellow, but there was no visual evidence of turbidity or precipitation of the salts.

Comparative

**Example 4: Method (A) preparation of liposomal Dutogliptin formulation with Dutogliptin free base**

The protocol of the Comparison Example can be used with the modification that Dutogliptin tartrate salt is replaced by the free base form of Dutogliptin.

## Claims

1. A method for the preparation of a liposomal Dutogliptin formulation, comprising the steps:
(B)

B.1) providing an organic phase comprising

(a) one or more phospholipids, wherein the amount of said one or more phospholipids is between 3%(w/w) and 30%(w/w) based on the sum of (a), (b) and (c), preferably 22.36%(w/w) based on the sum of (a), (b) and (c);
(b) one or more organic solvents selected from the group consisting of anisole, ethylacetate, 1,4-dioxane, dimethylcarbonate, dimethylsulfoxide, glycofurol, N,N-dimethylacetamide, N,N-dimethylformamide, N-methyl-2-pyrrolidone (NMP), isopropylideneglycerol, 1-butanol, 2-butanol and tert-butanol or a combination of any of the foregoing; wherein the amount of the said one or more organic solvents is between 60%(w/w) and 97%(w/w) based on the sum of (a), (b) and (c), preferably 74.43%(w/w) based on the sum of (a), (b) and (c);
(c) optionally one or more further organic component(s), which are neither (a) nor (b), wherein the amount of said one or more further organic component(s) is at most 10% based on the sum of (a), (b) and (c);

wherein the sum of (a), (b) and (c) sums up to 100%; and
B.2) providing an aqueous phase comprising

(d) an aqueous medium, wherein the amount of the aqueous medium is between 66%(w/w) and 90%(w/w) based on the sum of (d), (e) and (f), preferably 84%(w/w) based on the sum of (d), (e) and (f);
(e) optionally one or more further components selected from the group consisting of a buffer system, NaOH, and HCl, wherein the amount of all further components is at most 2%(w/w) based on the sum of (d), (e) and (f);
(f) optionally a bulking agent, preferably trehalose, wherein the amount of the bulking agent is at most 10% (w/w) based on the sum of (d), (e) and (f), preferably between 3%(w/w) and 7%(w/w) based on the sum of (d), (e) and (f);

wherein the sum of (d), (e) and (f) sums up to 100%; and
B.3) mixing the aqueous phase and the organic phase until a ratio between 3:1 and 1:3, preferably of 1:1, aqueous phase to organic phase is reached resulting in a nano-dispersed system;
B.4) lyophilize the nano-disperse system resulting in a lyophilizate;
B.5) reconstitute the lyophilizate of step B.4 with an aqueous solution resulting in a liposomal Dutogliptin formulation, preferably wherein the final concentration of Dutogliptin in the liposomal Dutogliptin formulation is between 25 mg/ml and 60 mg/ml, wherein the aqueous solution comprises

(i) 67%(w/w) and 100%(w/w) of an aqueous medium based on the total weight of the aqueous solution, optionally further comprising a tonicity adjusting agent, wherein the amount of the tonicity adjusting agent is at most 20%(w/w) based on the total weight of the aqueous solution optionally further comprising a buffer system, and
(ii) Dutogliptin, preferably in form of its tartrate salt or in form of its free base form, wherein the amount of Dutogliptin is between 5%(w/w) and 13%(w/w), more preferably 10$\pm$0.5)%(w/w) based on the total weight of the aqueous solution when the Dutogliptin is present in its tartrate salt form or free base form; or (6$\pm$0.4)% (w/w) based on the total weight of the aqueous solution when the Dutogliptin is present in its free base form

2. The method according to claim 1, wherein

i) the organic solvent is tert-butanol (TBA) and/or ii) the organic phase further comprises cholesterol and wherein

the amount of cholesterol is between 2.5%(w/w) and 4%(w/w), preferably 3.2%(w/w).

**3.** The method according to any of the preceding claims, wherein the organic phase comprises as a phospholipid lecithin and wherein the amount of lecithin is between 18.1%(w/w) and 26.2%(w/w) more preferably 22.13%(w/w).

**4.** The method according to any of the preceding claims, wherein the organic phase further comprises as phospholipid(s) phosphatidylglycerol (PG), preferably 1,2-Dioleoyl-sn-glycero-3-phosphoglycerol sodium salt (DOPG-Na), wherein the total amount of PG is between 0.1%(w/w) and 0.4%(w/w), preferably 0.23%(w/w).

**5.** The method according to any of the preceding claims, wherein the pH of the aqueous phase is between 7 and 7.8, preferably 7.4.

**6.** The method according to any of the preceding claims, wherein
the D90 of the liposomes of the liposomal Dutogliptin formulation resulting from the reconstitution step is between 1 μm and 4,5 μm.

**7.** The method according to any of the preceding claims, wherein the only solvent of an aqueous solution is water.

**8.** The method according to any of the preceding claims, wherein the aqueous solution is an aqueous NaCl solution wherein the amount of NaCl is between 8g/l and 10 g/l, preferably between 8.8 g/l and 9.2 g/l, more preferably 9 g/l.

**9.** The method according to any of the preceding claims, wherein the aqueous solution in Step B.5 is a Dutogliptin tartrate solution.

**10.** The method according to claim 9, wherein the aqueous solution further comprises 9 g/l NaCl.

**11.** The method according to any of the preceding claims, wherein the total amount of Dutogliptin in the liposomal Dutogliptin formulation is between 60 mg and 100 mg.

**12.** A liposomal Dutogliptin formulation comprising

(i) an aqueous medium, preferably water, wherein the amount of the aqueous medium is between 65%(w/w) and 90%(w/w) based on the total weight of the liposomal Dutogliptin formulation;
(ii) a lipid, preferably cholesterol, wherein the amount of said lipid, preferably cholesterol is between 1%(w/w) and 2.5%(w/w) based on the total weight of the liposomal Dutogliptin formulation;
(iii) one or more phospholipid(s), wherein the amount of said one or more phospholipid(s) is between 7%(w/w) and 15%(w/w), preferably (11.2±0.4)(w/w), based on the total weight of the liposomal Dutogliptin formulation, preferably, wherein the one or more phospholipids are phosphatidylcholine (PC) , phosphatidylglycerol (PG) and 1,2-distearoyl-*sn*-glycero-3-[phospho(1'-*rac*-glycerol)] (DSPG);
(iv) one or more agent(s) selected from the group consisting of glycine, arginine, proline, or any other amino acid known to be suitable as a bulking agent, a saccharide component selected from the group consisting of sucrose, trehalose, arabinose, erythritol, fructose, galactose, glucose, lactose, maltitol, maltose, maltotriose, mannitol, mannobiose, mannose, ribose, sorbitol, xylitol, xylose, dextran, dextrose, and NaCl, wherein the total amount of said one or more agent(s) is between 1.5%(w/w) and 5.5%(w/w);
(v) Dutogliptin, wherein the amount of Dutogliptin, preferably in its tartrate salt form or free base form, is between 3%(w/w) and 12%(w/w), preferably (5.3±0.3) (w/w) based on the total weight of the liposomal Dutogliptin formulation and the final concentration of Dutogliptin in the liposomal Dutogliptin formulation is between 25 mg/ml and 60 mg/ml;

wherein the sum of (i) to (v) sums up to 100%.

**13.** The liposomal Dutogliptin formulation according to claim 12, wherein said liposomal Dutogliptin formulation is lyophilized.

**14.** A pharmaceutical composition comprising the liposomal Dutogliptin formulation according to claim 12 or 13.

**15.** A kit comprising a lyophilizate described in claims 1 to 5 in a container and a pharmaceutical aqueous solution described in claim 1, 7, 8 or 10 in a second container.

16. The kit according to claim 15, further comprising a manual how to combine the content of the first and second container to receive a liposomal Dutogliptin formulation according to claim 12 or 13.

## Patentansprüche

1. Verfahren zur Herstellung einer liposomalen Dutogliptin-Formulierung, umfassend die Schritte:
(B)

B.1) Bereitstellen einer organischen Phase, umfassend

(a) ein oder mehrere Phospholipide, wobei die Menge des einen oder der mehreren Phospholipide basierend auf der Summe von (a), (b) und (c) zwischen 3 Gew.-% und 30 Gew.-%, vorzugsweise basierend auf der Summe von (a), (b) und (c) 22,36 Gew.-% beträgt;
(b) ein oder mehrere organische Lösungsmittel, ausgewählt aus der Gruppe bestehend aus Anisol, Ethylacetat, 1,4-Dioxan, Dimethylcarbonat, Dimethylsulfoxid, Glykofurol, N,N-Dimethylacetamid, N,N-Dimethylformamid, N-Methyl-2-pyrrolidon (NMP), Isopropylidenglycerin, 1-Butanol, 2-Butanol und tert-Butanol oder einer Kombination aus beliebigen der vorstehenden; wobei die Menge des einen oder der mehreren organischen Lösungsmittel basierend auf der Summe von (a), (b) und (c) zwischen 60 Gew.-% und 97 Gew.-%, vorzugsweise basierend auf der Summe von (a), (b) und (c) 74,43 Gew.-% beträgt;
(c) gegebenenfalls eine oder mehrere weitere organische Komponente(n), die weder (a) noch (b) sind, wobei die Menge der einen oder mehreren weiteren organischen Komponente(n) höchstens 10 % beträgt, basierend auf der Summe von (a), (b) und (c);

wobei die Summe von (a), (b) und (c) 100 % ergibt; und
B.2) Bereitstellen einer wässrigen Phase, umfassend

(d) ein wässriges Medium, wobei die Menge des wässrigen Mediums basierend auf der Summe von (d), (e) und (f) zwischen 66 Gew.-% und 90 Gew.-%, vorzugsweise basierend auf der Summe von (d), (e) und (f) 84 Gew.-% beträgt;
(e) gegebenenfalls eine oder mehrere weitere Komponenten, ausgewählt aus der Gruppe bestehend aus einem Puffersystem, NaOH und HCl, wobei die Menge aller weiteren Komponenten basierend auf der Summe von (d), (e) und (f) höchstens 2 Gew.-% beträgt;
(f) gegebenenfalls einen Füllstoff, vorzugsweise Trehalose, wobei die Menge des Füllstoffs basierend auf der Summe von (d), (e) und (f) höchstens 10 Gew.-%, vorzugsweise basierend auf der Summe von (d), (e) und (f) zwischen 3 Gew.-% und 7 Gew.-% beträgt;

wobei die Summe von (d), (e) und (f) 100 % ergibt; und
B.3) Mischen der wässrigen Phase und der organischen Phase, bis ein Verhältnis zwischen 3:1 und 1:3, vorzugsweise 1:1 von wässriger Phase und organischer Phase erreicht ist, was zu einem nanodispersen System führt;
B.4) Lyophilisieren des nanodispersen Systems, was zu einem Lyophilisat führt;
B.5) Rekonstituieren des Lyophilisats aus Schritt B.4 mit einer wässrigen Lösung, was zu einer liposomalen Dutogliptin-Formulierung führt, vorzugsweise wobei die Endkonzentration von Dutogliptin in der liposomalen Dutogliptin-Formulierung zwischen 25 mg/ml und 60 mg/ml beträgt, wobei die wässrige Lösung Folgendes umfasst:

(i) basierend auf dem Gesamtgewicht der wässrigen Lösung 67 Gew.-% und 100 Gew.-% eines wässrigen Mediums, gegebenenfalls ferner umfassend ein Tonizität einstellendes Mittel, wobei die Menge des Tonizität einstellenden Mittels höchstens 20 Gew.-% beträgt, basierend auf dem Gesamtgewicht der wässrigen Lösung, die gegebenenfalls ferner ein Puffersystem umfasst, und
(ii) Dutogliptin, vorzugsweise in Form seines Tartratsalzes oder in Form seiner freien Base, wobei die Menge an Dutogliptin zwischen 5 Gew.-% und 13 Gew.-%, mehr bevorzugt (10 $\pm$ 0,5) Gew.-% basierend auf dem Gesamtgewicht der wässrigen Lösung beträgt, wenn das Dutogliptin in seiner Tartratsalzform oder freien Basenform vorliegt; oder (6 $\pm$ 0,4) Gew.-% basierend auf dem Gesamtgewicht der wässrigen Lösung, wenn das Dutogliptin in seiner freien Basenform vorliegt

2. Verfahren nach Anspruch 1, wobei

i) das organische Lösungsmittel tert-Butanol (TBA) ist und/oder ii) die organische Phase ferner Cholesterin umfasst und wobei die Menge an Cholesterin zwischen 2,5 Gew.-% und 4 Gew.-%, vorzugsweise 3,2 Gew.-% beträgt.

**3.** Verfahren nach einem der vorhergehenden Ansprüche, wobei die organische Phase als Phospholipid Lecithin umfasst und wobei die Menge an Lecithin zwischen 18,1 Gew.-% und 26,2 Gew.-%, mehr bevorzugt 22,13 Gew.-% beträgt.

**4.** Verfahren nach einem der vorhergehenden Ansprüche, wobei die organische Phase ferner als Phospholipid(e) Phosphatidylglycerin (PG) umfasst, vorzugsweise 1,2-Dioleoyl-sn-glycero-3-phosphoglycerin, Natriumsalz (DOPG-Na), wobei die Gesamtmenge an PG zwischen 0,1 Gew.-% und 0,4 Gew.-%, vorzugsweise 0,23 Gew.-% beträgt.

**5.** Verfahren nach einem der vorhergehenden Ansprüche, wobei der pH-Wert der wässrigen Phase zwischen 7 und 7,8, vorzugsweise bei 7,4 liegt.

**6.** Verfahren nach einem der vorhergehenden Ansprüche, wobei
die D90 der Liposomen der liposomalen Dutogliptin-Formulierung, die aus dem Rekonstitutionsschritt resultiert, zwischen 1 $\mu$m und 4,5 $\mu$m beträgt.

**7.** Verfahren nach einem der vorhergehenden Ansprüche, wobei das einzige Lösungsmittel einer wässrigen Lösung Wasser ist.

**8.** Verfahren nach einem der vorhergehenden Ansprüche, wobei die wässrige Lösung eine wässrige NaCl-Lösung ist, wobei die Menge an NaCl zwischen 8 g/l und 10 g/l, vorzugsweise zwischen 8,8 g/l und 9,2 g/l, mehr bevorzugt bei 9 g/l liegt.

**9.** Verfahren nach einem der vorhergehenden Ansprüche, wobei die wässrige Lösung in Schritt B.5 eine Dutogliptintartrat-Lösung ist.

**10.** Verfahren nach Anspruch 9, wobei die wässrige Lösung ferner 9 g/l NaCl umfasst.

**11.** Verfahren nach einem der vorhergehenden Ansprüche, wobei die Gesamtmenge an Dutogliptin in der liposomalen Dutogliptin-Formulierung zwischen 60 mg und 100 mg liegt.

**12.** Liposomale Dutogliptin-Formulierung, umfassend

(i) ein wässriges Medium, vorzugsweise Wasser, wobei die Menge des wässrigen Mediums basierend auf dem Gesamtgewicht der liposomalen Dutogliptin-Formulierung zwischen 65 Gew.-% und 90 Gew.-% beträgt;
(ii) ein Lipid, vorzugsweise Cholesterin, wobei die Menge an Lipid, vorzugsweise an Cholesterin, basierend auf dem Gesamtgewicht der liposomalen Dutogliptin-Formulierung zwischen 1 Gew.-% und 2,5 Gew.-% beträgt;
(iii) ein oder mehrere Phospholipid(e), wobei die Menge des einen oder der mehreren Phospholipid(e) basierend auf dem Gesamtgewicht der liposomalen Dutogliptin-Formulierung zwischen 7 Gew.-% und 15 Gew.-%, vorzugsweise (11,2 $\pm$ 0,4) Gew.-% beträgt; vorzugsweise wobei das eine oder die mehreren Phospholipide Phosphatidylcholin (PC), Phosphatidylglycerin (PG) und 1,2-Distearoyl-*sn*-glycero-3-[phospho(1'-*rac*-glycerin)] (DSPG) sind;
(iv) ein oder mehrere Mittel, ausgewählt aus der Gruppe bestehend aus Glycin, Arginin, Prolin oder jeder anderen Aminosäure, von der bekannt ist, dass sie als Füllstoff geeignet ist, einer Saccharidkomponente, ausgewählt aus der Gruppe bestehend aus Saccharose, Trehalose, Arabinose, Erythrit, Fruktose, Galaktose, Glukose, Laktose, Maltit, Maltose, Maltotriose, Mannit, Mannobiose, Mannose, Ribose, Sorbit, Xylit, Xylose, Dextran, Dextrose, und NaCl, wobei die Gesamtmenge des einen oder mehrerer Mittel zwischen 1,5 Gew.-% und 5,5 Gew.-% beträgt;
(v) Dutogliptin, wobei die Menge an Dutogliptin, vorzugsweise in seiner Tartratsalzform oder freien Basenform, basierend auf dem Gesamtgewicht der liposomalen Dutogliptin-Formulierung zwischen 3 Gew.-% und 12 Gew.-%, vorzugsweise (5,3 $\pm$ 0,3) Gew.-% beträgt und die Endkonzentration von Dutogliptin in der liposomalen Dutogliptin-Formulierung zwischen 25 mg/ml und 60 mg/ml liegt;

wobei die Summe von (i) bis (v) 100 % ergibt.

**13.** Liposomale Dutogliptin-Formulierung nach Anspruch 12, wobei die liposomale Dutogliptin-Formulierung lyophilisiert

ist.

**14.** Pharmazeutische Zusammensetzung, umfassend die liposomale Dutogliptin-Formulierung nach Anspruch 12 oder 13.

**15.** Kit, umfassend ein Lyophilisat nach den Ansprüchen 1 bis 5 in einem Behälter und eine pharmazeutische wässrige Lösung nach Anspruch 1, 7, 8 oder 10 in einem zweiten Behälter.

**16.** Kit nach Anspruch 15, ferner umfassend eine Anleitung, wie der Inhalt des ersten und zweiten Behälters zu kombinieren ist, um eine liposomale Dutogliptin-Formulierung nach Anspruch 12 oder 13 zu erhalten.

**Revendications**

**1.** Procédé de préparation d'une formulation liposomale de dutogliptine, comprenant les étapes suivantes :
(B)

B.1) la fourniture d'une phase organique comprenant

(a) un ou plusieurs phospholipides, la quantité desdits un ou plusieurs phospholipides étant comprise entre 3 % (p/p) et 30 % (p/p) par rapport à la somme de (a), (b) et (c), de préférence 22,36 % (p/p) par rapport à la somme de (a), (b) et (c) ;
(b) un ou plusieurs solvants organiques choisis dans le groupe constitué par l'anisole, l'acétate d'éthyle, le 1,4-dioxane, le carbonate de diméthyle, le diméthylsulfoxyde, le glycofurol, le N,N-diméthylacétamide, le N,N-diméthylformamide, la N-méthyl-2-pyrrolidone (NMP), l'isopropylidèneglycérol, le 1-butanol, le 2-butanol et le tert-butanol ou une association de quelconques des précédents ; la quantité desdits un ou plusieurs solvants organiques étant comprise entre 60 % (p/p) et 97 % (p/p) par rapport à la somme de (a), (b) et (c), de préférence 74,43 % (p/p) par rapport à la somme de (a), (b) et (c) ;
(c) éventuellement un ou plusieurs autres composants organiques, qui ne sont ni (a) ni (b), la quantité desdits un ou plusieurs autres composants organiques étant au plus de 10 % par rapport à la somme de (a), (b) et (c) ;

la somme de (a), (b) et (c) étant de 100 % ; et
B.2) la fourniture d'une phase aqueuse comprenant

(d) un milieu aqueux, la quantité du milieu aqueux étant comprise entre 66 % (p/p) et 90 % (p/p) sur la base de la somme de (d), (e) et (f), de préférence 84 % (p/p) sur la base de la somme de (d), (e) et (f) ;
(e) éventuellement un ou plusieurs autres composants choisis dans le groupe constitué par un système tampon, NaOH et HCl, la quantité de tous les autres composants étant au plus de 2 % (p/p) par rapport à la somme de (d), (e) et (f) ;
(f) éventuellement un agent de charge, de préférence le tréhalose, la quantité de l'agent de charge étant au plus de 10 % (p/p) par rapport à la somme de (d), (e) et (f), de préférence entre 3 % (p/p) et 7 % (p/p) par rapport à la somme de (d), (e) et (f) ;

la somme de (d), (e) et (f) étant de 100 % ; et
B.3) le mélange de la phase aqueuse et de la phase organique jusqu'à ce qu'un rapport de phase aqueuse à phase organique soit compris entre 3:1 et 1:3, de préférence égal à 1:1, pour obtenir un système nanodispersé ;
B.4) la lyophilisation du système nanodispersé pour obtenir un lyophilisat ;
B.5) la reconstitution du lyophilisat de l'étape B.4 avec une solution aqueuse pour obtenir une formulation liposomale de dutogliptine, de préférence la concentration finale de dutogliptine dans la formulation liposomale de dutogliptine étant comprise entre 25 mg/ml et 60 mg/ml, la solution aqueuse comprenant

(i) 67 % (p/p) et 100 % (p/p) d'un milieu aqueux par rapport au poids total de la solution aqueuse, comprenant éventuellement en outre un agent d'ajustement de la tonicité, la quantité de l'agent d'ajustement de la tonicité étant d'au plus 20 % (p/p) par rapport au poids total de la solution aqueuse comprenant éventuellement en outre un système tampon, et
(ii) de la dutogliptine, de préférence sous forme de son sel de tartrate ou sous forme de sa forme de base libre, la quantité de dutogliptine étant comprise entre 5 % (p/p) et 13 % (p/p), plus préférablement 10 $\pm$ 0,5 %(p/p) par rapport au poids total de la solution aqueuse lorsque la dutogliptine est présente sous sa forme sel de

tartrate ou sa forme base libre ; ou (6±0,4) % (p/p) par rapport au poids total de la solution aqueuse lorsque la dutogliptine est présente sous sa forme base libre

**2.** Procédé selon la revendication 1, dans lequel

i) le solvant organique est le tert-butanol (TBA) et/ou ii) la phase organique comprend en outre du cholestérol et dans lequel la quantité de cholestérol est comprise entre 2,5 % (p/p) et 4 % (p/p), de préférence 3,2 % (p/p).

**3.** Procédé selon l'une quelconque des revendications précédentes, dans lequel la phase organique comprend de la lécithine en tant que phospholipide et dans lequel la quantité de lécithine est comprise entre 18,1 % (p/p) et 26,2 % (p/p), plus préférablement 22,13 % (p/p).

**4.** Procédé selon l'une quelconque des revendications précédentes, dans lequel la phase organique comprend en outre, en tant que phospholipide(s), du phosphatidylglycérol (PG), de préférence du 1,2-dioléoyl-sn-glycéro-3-phosphoglycérol, sel de sodium (DOPG-Na), la quantité totale de PG étant comprise entre 0,1 % (p/p) et 0, 4 % (p/p), de préférence 0,23 % (p/p).

**5.** Procédé selon l'une quelconque des revendications précédentes, dans lequel le pH de la phase aqueuse est compris entre 7 et 7,8, de préférence 7,4.

**6.** Procédé selon l'une quelconque des revendications précédentes, dans lequel le D90 des liposomes de la formulation liposomale de dutogliptine résultant de l'étape de reconstitution est compris entre 1 μm et 4,5 μm.

**7.** Procédé selon l'une quelconque des revendications précédentes, dans lequel le seul solvant d'une solution aqueuse est l'eau.

**8.** Procédé selon l'une quelconque des revendications précédentes, dans lequel la solution aqueuse est une solution aqueuse de NaCl dans laquelle la quantité de NaCl est comprise entre 8 g/l et 10 g/l, de préférence entre 8,8 g/l et 9,2 g/l, plus préférablement 9 g/l.

**9.** Procédé selon l'une quelconque des revendications précédentes, dans lequel la solution aqueuse dans l'étape B.5 est une solution de tartrate de dutogliptine.

**10.** Procédé selon la revendication 9, dans lequel la solution aqueuse comprend en outre 9 g/l de NaCl.

**11.** Procédé selon l'une quelconque des revendications précédentes, dans lequel la quantité totale de dutogliptine dans la formulation liposomale de dutogliptine est comprise entre 60 mg et 100 mg.

**12.** Formulation liposomale de dutogliptine comprenant

(i) un milieu aqueux, de préférence de l'eau, la quantité du milieu aqueux étant comprise entre 65 % (p/p) et 90 % (p/p) par rapport au poids total de la formulation liposomale de dutogliptine ;
(ii) un lipide, de préférence du cholestérol, la quantité dudit lipide, de préférence du cholestérol, étant comprise entre 1 % (p/p) et 2,5 % (p/p) par rapport au poids total de la formulation liposomale de dutogliptine ;
(iii) un ou plusieurs phospholipides, la quantité desdits un ou plusieurs phospholipides étant comprise entre 7 % (p/p) et 15 % (p/p), de préférence (11,2 ± 0,4) (p/p), sur la base du poids total de la formulation liposomale de dutogliptine, de préférence, les un ou plusieurs phospholipides étant la phosphatidylcholine (PC), le phosphatidylglycérol (PG) et le 1,2-distéaroyl-sn-glycéro-3-[phospho(1'-rac-glycérol)] (DSPG) ;
(iv) un ou plusieurs agents choisis dans le groupe constitué par la glycine, l'arginine, la proline ou tout autre acide aminé connu pour être approprié en tant qu'agent de charge, un composant saccharide choisi dans le groupe constitué par le saccharose, le tréhalose, l'arabinose, l'érythritol, le fructose, le galactose, le glucose, le lactose, le maltitol, le maltose, le maltotriose, le mannitol, le mannobiose, le mannose, le ribose, le sorbitol, le xylitol, le xylose, le dextrane, le dextrose et NaCl, la quantité totale desdits un ou plusieurs agent(s) étant comprise entre 1,5 % (p/p) et 5,5 % (p/p) ;
(v) de la dutogliptine, la quantité de dutogliptine, de préférence sous sa forme sel de tartrate ou sa forme base libre, étant comprise entre 3 % (p/p) et 12 % (p/p), de préférence (5,3 ± 0,3) (p/p) par rapport au poids total de la formulation liposomale de dutogliptine et la concentration finale de dutogliptine dans la formulation liposomale de dutogliptine étant comprise entre 25 mg/ml et 60 mg/ml ;

la somme de (i) à (v) étant de 100 %).

**13.** Formulation liposomale de dutogliptine selon la revendication 12, dans laquelle ladite formulation liposomale de dutogliptine est lyophilisée.

**14.** Composition pharmaceutique comprenant la formulation liposomale de dutogliptine selon la revendication 12 ou 13.

**15.** Kit comprenant un lyophilisat selon les revendications 1 à 5 dans un récipient et une solution aqueuse pharmaceutique selon la revendication 1, 7, 8 ou 10 dans un deuxième récipient.

**16.** Kit selon la revendication 15, comprenant en outre un manuel expliquant comment combiner le contenu du premier et du deuxième récipient pour obtenir une formulation liposomale de dutogliptine selon la revendication 12 ou 13.

Figure 1

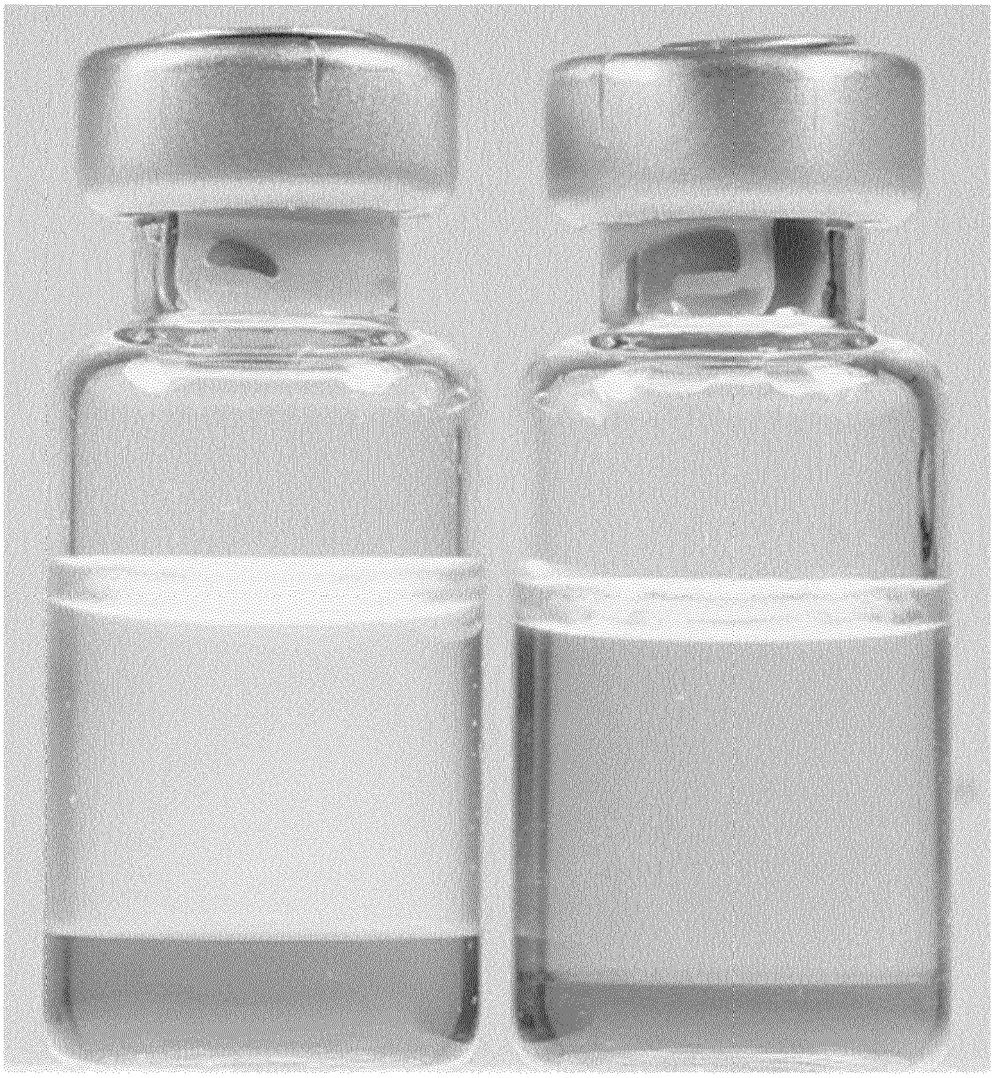

Figure 2

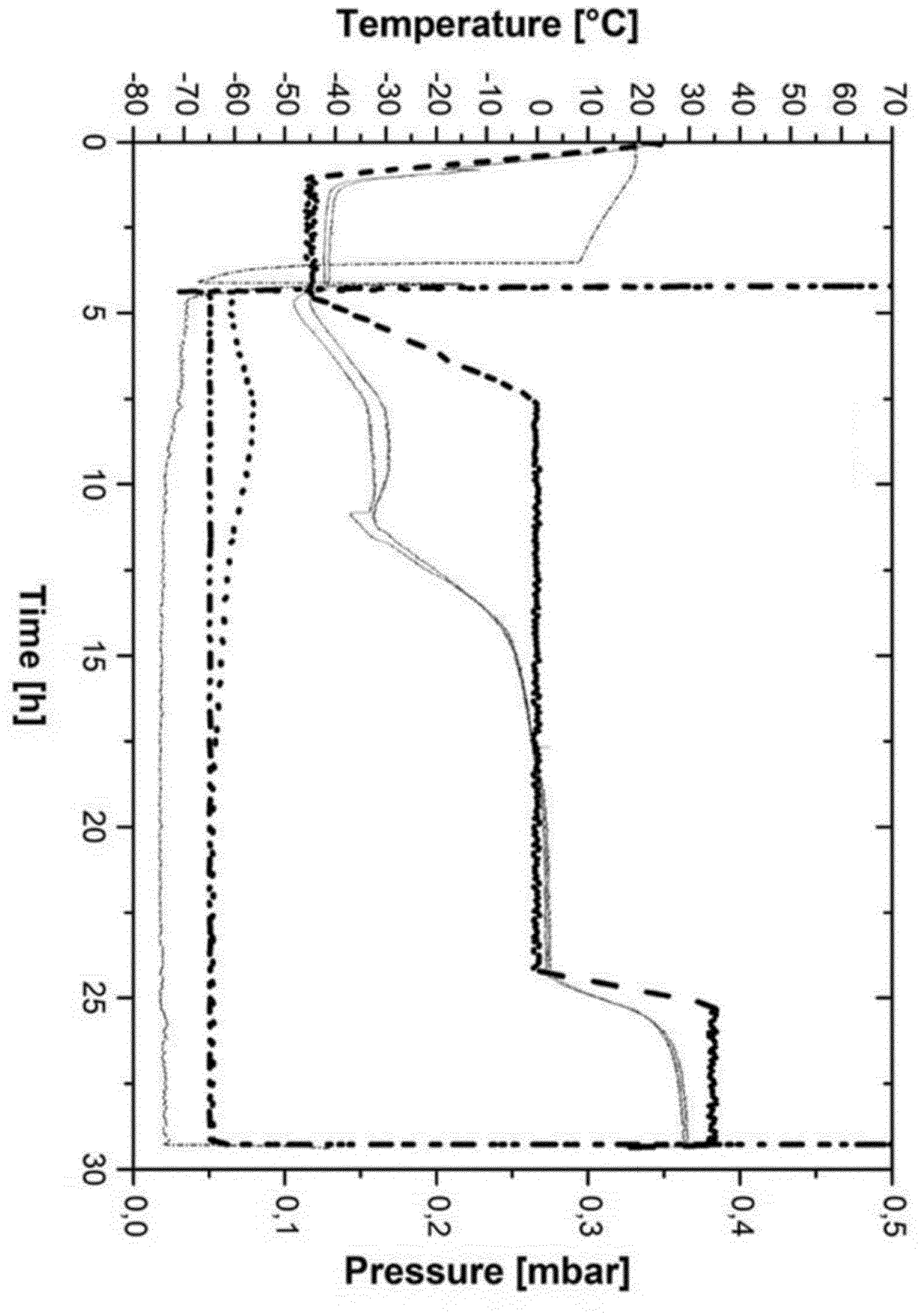
Temperature [°C]
-80 -70 -60 -50 -40 -30 -20 -10 0 10 20 30 40 50 60 70
Time [h]
0 5 10 15 20 25 30
Pressure [mbar]
0,0 0,1 0,2 0,3 0,4 0,5

Figure 3

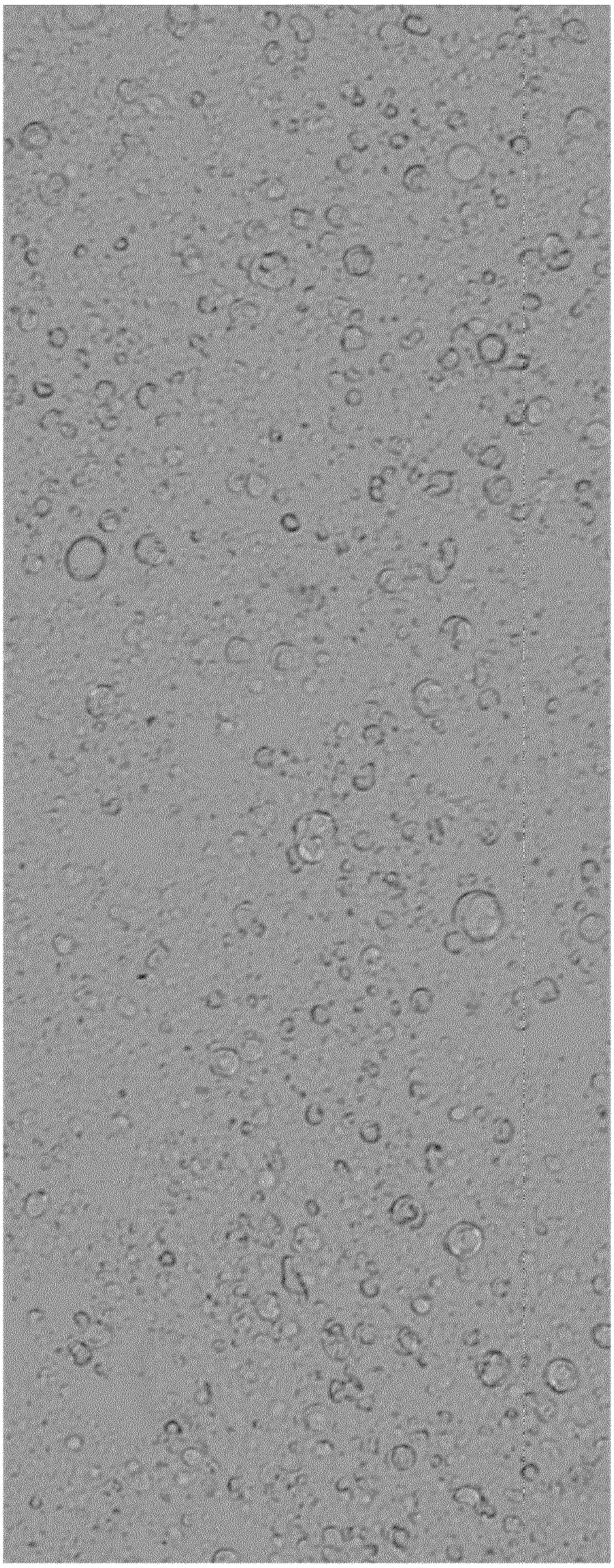

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3263134 A


### Non-patent literature cited in the description


- *CHEMICAL ABSTRACTS*, 852329-66-9
- *CHEMICAL ABSTRACTS*, 57-88-5
- *CHEMICAL ABSTRACTS*, 100-66-3
- *CHEMICAL ABSTRACTS*, 141-78-6
- *CHEMICAL ABSTRACTS*, 123-91-1
- *CHEMICAL ABSTRACTS*, 616-38-6
- *CHEMICAL ABSTRACTS*, 67-68-5
- *CHEMICAL ABSTRACTS*, 31692-85-0
- *CHEMICAL ABSTRACTS*, 127-19-5
- *CHEMICAL ABSTRACTS*, 68-12-2
- *CHEMICAL ABSTRACTS*, 872-50-4
- *CHEMICAL ABSTRACTS*, 100-79-8
- *CHEMICAL ABSTRACTS*, 71-36-3
- *CHEMICAL ABSTRACTS*, 78-92-2
- *CHEMICAL ABSTRACTS*, 75-65-0